# EUROPEAN PATENT APPLICATION

(11) **EP 3 153 176 A1**
(43) Date of publication of application: **12.04.2017**
(21) Application number: 16188862.3
(22) Date of filing: 27.10.2011
(51) Int. Cl.: A61K 39/395, A61K 39/00, A61P 25/28, A61K 39/39, A61K 31/00, A61K 31/7008, A61K 31/7056

(54) **IMMUNOGENIC COMPOSITIONS AND METHODS FOR TREATING NEUROLOGIC DISORDERS**

(30) Priority: 27.10.2010 US 407235 P; 26.01.2011 GB 201101331
(62) Divisional of application: 11776775.6
(71) Applicant: GlaxoSmithKline Biologicals S.A., 1330 Rixensart (BE)
(72) Inventor: HALLE, Maxime, Laval, Québec H7V 3S8 (CA); LAROCQUE, Daniel, Laval, Québec H7V 3S8 (CA); PALMANTIER, Remi, Laval, Québec H7V 3S8 (CA); PRIEELS, Jean-Paul, 1330 Rixensart (BE); TRIBOUT-JOVER, Pascale, Laval, Québec H7V 3S8 (CA)
(74) Representative: Thornley, Rachel Mary

(57) **Abstract**

Compositions and methods for preventing and/or reducing amyloid deposition in a subject comprising treatment of a subject with a composition comprising a TLR4 agonist free of endotoxin are provided. Also provided is a TLR4 agonist free of endotoxin for preventing and/or reducing Alzheimer's disease. Pharmaceutical compositions consisting of, or consisting essentially of, an aminoalkyl glucosaminide phosphate, 3D-MPL, AS01 B or an AGP in combination with an oil in water emulsion are also provided.

## Description

### Field of the Invention

The present invention relates to preventing and treating Amyloid-[beta] deposition, and/or Alzheimer's disease, and compositions for the same.

### Alzheimer's disease (AD)

Alzheimer's disease (AD) is a neurodegenerative disorder that represents the most important cause of dementia in humans. Extracellular deposits of β-amyloid peptides (Aβ), often termed senile plaques, and formation of intracellular neurofibrillary tangles of hyperphosphorylated tau protein are the two principal hallmarks of this disease. Aβ aggregates are known to induce synaptic dysfunction, long term potential reduction in the hippocampus region of the brain and thus, are linked with learning and memory deficits both in human and in mouse models of AD, making Aβ deposits a target for prevention or treatment against this disorder.

AD has been observed in all races and ethnic groups worldwide and presents a major present and future public health problem. As many as 4.5 million Americans suffer from AD. The disease usually begins after age sixty, and risk goes up with age. While younger people also may get AD, it is much less common. About five percent of men and women aged sixty- five to seventy- four have AD, and nearly half of those age eighty-five and older may have the disease. It is important to note, however, that AD is not a normal part of aging. AD is at present incurable. No treatment that effectively prevents AD or reverses its symptoms or course is currently known.

The deposition of amyloid-beta (Abeta or Ab or Aβ herein) peptides in the central nervous system in the form of amyloid plaques is one of the hallmarks of AD (U.S. Patent Publication No. 20040214774 to Wisniewski et al; U.S. Patent No. 6,114,133 to Seubert; Wegiel et al., "Alzheimer Dementia Neuropathology," in Dementia: Presentations, Differential Diagnosis & Nosology, 89-120 (Emery & Oxman, eds., 2003). Several lines of evidence favour the conclusion that amyloid beta accumulation destroys neurons in the brain, leading to deficits in cognitive abilities. Because accumulation of amyloid beta appears to be the result of a shift in equilibrium from clearance toward deposition, identifying and promoting mechanisms that enhance amyloid beta clearance from the brain is highly desirable.

Intramuscular injection of Aβ₄₂ peptide in young transgenic mice, over expressing a mutated form of the human APP, efficiently prevented the formation of Aβ deposits, while its administration to older animals reduced their amyloid plaque burden. Schenk, D. et al. Nature 400, 173-177 (1999). Furthermore, the intracerebroventricular injection of a monoclonal antibody against Aβ efficiently prevented an injected Aβ oligomer from inhibiting long-term potentiation (an electrophysiological measurement correlating with memory), see Klyubin, I. et al. Nature medicine 11, 556-561 (2005). Thus, in the mouse model, vaccination is a valid approach in preventing AD-related phenotypes.

Vaccination was the first treatment approach which has been shown to have genuine impact on disease process, at least in animal models of AD (Sadowski et al., "Disease Modifying Approaches for Alzheimer's Pathology," Current Pharmaceutic Design, 13:1943-54 (2007); Wisniewski et al., "Therapeutic Approaches for Prion and Alzheimer's Diseases," FEBS J. 274:3784-98 (2007); Wisniewski et al., "Immunological and Anti-Chaperone Therapeutic Approaches for Alzheimer Disease," Brain Pathol. 15:72-77 (2005)). Vaccination of AD transgenic (Tg) mice with amyloid beta 1-42 or Abeta homologous peptides co-injected with Freund's adjuvant prevented the formation of amyloid beta deposition and as a consequence eliminate the behavioural impairments that are related to amyloid beta deposition (Schenk et al., "Immunization with Amyloid-Beta Attenuates Alzheimer-Disease-Like Pathology in the PDAPP Mouse," Nature 400:173-77 (1999); Sigurdsson et al., "Immunization with a Nontoxic/Nonfibrillar Amyloid-beta Homologous Peptide Reduces Alzheimer's Disease- Associated Pathology in Transgenic Mice," Am. J. Pathol. 159:439-47 (2001); Morgan et al., "A Beta Peptide Vaccination Prevents Memory Loss in an Animal Model of Alzheimer's Disease," Nature 408:982-85 (2001); Janus et al., "A Beta Peptide Immunization Reduces Behavioural Impairment and Plaques in a Model of Alzheimer's Disease," Nature 408:979-82 (2000)).

The striking biological effect of the vaccine in preclinical testing and the apparent lack of side effects in AD Tg mice encouraged Elan Pharmaceuticals, Inc./Wyeth Research to launch clinical trials with a vaccine designated as AN 1792 which contained pre-aggregated Abeta1-42 and QS21 as an adjuvant. It was thought that this type of vaccine design would induce a strong adaptive cell mediated immune response, because QS21 is known to be a strong inducer of T-helper type-1 (Th-I) lymphocytes. The phase II of the trial was prematurely terminated when 6% of vaccinated patients manifested symptoms of acute meningoencephalitis. An autopsy performed on one of the affected patients revealed an extensive cytotoxic T-cell reaction surrounding some cerebral blood vessels. Analysis of the A[beta] load in the brain cortex, however, suggested that Abeta clearance had occurred (Nicoll et al., "Neuropathology of Human Alzheimer Disease after Immunization with Amyloid- beta Peptide: A Case Report," Nature Med. 9:448-52 (2003)). Neuropsychiatric testing of vaccinated patients who mounted an immune response showed a modest but statistically significant cognitive benefit, demonstrating an improvement on some cognitive testing scales comparing to baseline and a slowed rate of disease progression in patients who had developed antibodies to Abeta (Hock et al., "Antibodies Against Beta-Amyloid Slow Cognitive Decline in Alzheimer's Disease," Neuron 38:547-54 (2003)). This indicated that the vaccination approach could be beneficial for human AD patients, but that the concept of the vaccine may need redesigning. WO2009105641 acknowledges the above background and is directed to a method of preventing or reducing amyloid deposition in a subject. This method involves selecting a subject with amyloid deposits and stimulating the innate immune system of the selected subject under conditions effective to reduce the amyloid deposits. In particular a TLR9 agonist is used.

Frenkel et al (Ann Neurol. 2008 May;63(5):591-601) disclose the use of a nasal proteosome adjuvant to prevent amyloid deposition.

The brain itself has an inherent immunological mechanism to prevent disease and toxic overload. Microglia are the immune cells of the brain, and like peripheral macrophages, they are phagocytes, produce cytokines, and participate in the innate immune response by protecting the brain against invading pathogens. In AD transgenic mice, Aβ stimulate the recruitment of blood-derived microglia, a cell lineage that is specifically capable of eliminating amyloid deposit by phagocytosis, see Simard, A.R. et al. Neuron 49, 489-502 (2006).. Also the intrahippocampal injection of lipopolysaccharide (LPS) induces the recruitment of activated bone marrow-derived microglia leading to the decreased burden of Aβ in a transgenic mouse model for AD, see Malm, T.M. et al. Neurobiol Dis 18, 134-142 (2005).

Furthermore, like LPS, Aβ can stimulate the expression of the Toll-like receptor 2 (TLR2) in microglia. Interestingly, the inactivation of the TLR2 gene in APP transgenic mice (APP Tg/TLR2^{-/-}) caused increased Aβ₄₂ production and accelerated cognitive impairment Richard, K.L. et al. J Neurosci 28, 5784-5793 (2008). Thus, the TLR2 pathway plays a critical role in the development of the AD pathologies.

Protollin injection in APP Tg mice significantly improved their memory and stimulated the activation of microglia, which correlated with a reduced Aβ burden [Frenkel, D. et al. The Journal of clinical investigation 115, 2423-2433 (2005); Frenkel, D. et al. Ann Neurol 63, 591-601 (2008)]. Moreover, no apparent toxicity was observed following Protollin treatment. Frenkel, D. et al. Ann Neurol 63, 591-601 (2008).

There is an urgent need for both a prophylactic and curative treatment for Alzheimer's disease (AD). Murine models have indicated that an immunotherapeutic or vaccination approach is feasible in developing a vaccine for AD [Maim, T.M. et al. Neurobiol Dis 18, 134-142 (2005)].

### SUMMARY OF THE INVENTION

The present invention relates to:
A method of preventing and/or reducing amyloid deposition in a subject comprising treatment of a subject with an effective amount of a composition comprising a TLR4 agonist free of endotoxin.

A composition comprising a TLR4 agonist free of endotoxin for use in preventing and/or reducing amyloid deposition in a subject.

Use of a TLR4 agonist free of endotoxin in the manufacture of a medicament for preventing and/or reducing amyloid deposition in a subject.

A method of preventing and/or reducing Alzheimer's disease in a subject comprising treatment of a subject with an effective amount of a composition comprising a TLR4 agonist free of endotoxin.

A composition comprising a TLR4 agonist free of endotoxin for use in preventing and/or reducing Alzheimer's disease.

Use of a composition comprising a TLR4 agonist free of endotoxin in the manufacture of a medicament for preventing and/or reducing Alzheimer's disease.

A method for prevention or reduction of amyloid deposition and/ or Alzheimer's disease as described in all previous aspects of the invention using a pharmaceutical composition consisting of, or consisting essentially of, an AGP, 3D MPL, AS01 b, or an AGP in combination with an oil in water emulsion.

A pharmaceutical composition consisting, or consisting essentially of, a TLR4 agonist free of endotoxin and a pharmaceutically acceptable excipient.

A pharmaceutical composition consisting, or consisting essentially of, 3D MPL and a pharmaceutically acceptable excipient.

A pharmaceutical composition consisting, or consisting essentially of, MPL and a pharmaceutically acceptable excipient.

A pharmaceutical composition consisting, or consisting essentially of AS01 B and a pharmaceutically acceptable excipient.

A pharmaceutical composition consisting, or consisting essentially of an AGP and a pharmaceutically acceptable excipient.

### Figures

Figure 1: TLR2 mRNA transcription following injection of TLR4 agonists free of endotoxin.
Figure 2: Inflammatory cytokine (TNFα) in mouse sera following (2hr post injection) peripheral injection of TLR4 agonists free of endotoxin.
Figure 3: TLR4 agonists free of endotoxin trigger a higher number of CD11b + monocytes within the periphery.
Figure 4: Peripheral blood monocyte number following a single intramuscular injection of different doses of 3D MPL (5µg, 25µg and 50µg).
Figure 5: Peripheral blood monocyte number following a single intramuscular injection of different doses of AS01 B (1/20 vs 1/5 vs mouse full dose).
Figure 6: Peripheral blood monocyte number following a single intramuscular injection of different doses of CRX601 (0.2 µg to 20 µg).
Figure 7: Peripheral blood monocyte number following a single intramuscular injection of different doses of CRX601 (0.2 µg to 20 µg) in combination of constant dose of AS03.
Figure 8: Aβ42 *ex vivo* uptake by peripheral blood monocytes from adjuvanted mice.
Figure 9: Aβ total plaque loading analyses.
Figure 10: Twelve weekly injections of 3D-MPL or CRX527 or CRX601 or AS01 B in APP/PS1 mouse model shows a spatial memory improvement compared to non treated mice.
Figure 11: Passive avoidance retention test.
Figure 12: 3D Histology of the brain Ab plaque following 3D MPL and LPS treated APP/PS1 mice.
Figure 13: Results of behavioural analysis using TLR4 agonists free of endotoxin.
Figure 14: Reduction of monomeric Aβ in extra-cellular enriched fractions from brains of 3D MPL-injected mice.
Figure 15: Phagocytosis of beta-amyloid 1-42 peptide by human microglial cells after treatment with TLR4 agonists free of endotoxin
Figure 16 Representative picture of fluorescence microscopy of human microglia cell line showing the localization of Aβ1-42 within the lysosome after AS01 B treatment.
Figure 17: Innate cytokines profile from sera after either LPS or 3DMPL injected mice using the intraperitoneal route at 2hr or 6hr post injection time point. Results are shown in relative units (RU or pg/ml) of various cytokines/chemokines in sera for PBS and LPS or 3DMPL injected mice after 2 hours or 6 hours. N = 5 mice per group. The bars represent mean ± SEM; * P < 0.05, significantly different from compared groups. Tukey post-hoc test used for the comparison following ANOVA-1 analysis.
Figure 18: Innate cytokines profile from sera after either AS01B, AS03 or AS04D injected mice using the intramuscular at 2hr or 6hr post injection time point. Results are shown in relative units (RU or pg/ml) of various cytokines/chemokines in sera for PBS and LPS or 3D MPL injected mice after 2 hours or 6 hours. N = 5 mice per group. The bars represent mean ± SEM.
Figure 19: The number of monocytes is up-regulated 4.5 fold by AS01 B.
Figure 20: AS01B and QS21 + liposome stimulate *in vivo* an increase in monocyte number increase (panel A) and the monocyte activation state (Ly6C high) (panel B) most significantly after 24hrs in the C57BL/6 mouse peripheral blood following intra muscular injection.
Figure 21: AS01B and QS21 + liposome stimulate the *ex vivo* Aβ uptake by mouse peripheral blood monocytes most significantly after 24 hrs of the intra muscular injection in the C57BL/6 mouse.

### (In the Figures, all references to MPL are references to 3D-MPL)

### Detailed description

The invention generally relates to a method of preventing and/or reducing amyloid deposition or Alzheimer's disease, in a subject comprising treatment of a subject with a composition comprising a TLR4 agonist free of endotoxin, for example comprising an aminoalkyl glucosaminide phosphate (AGP), 3D-MPL or MPL.

As used herein, "amyloid" encompasses any insoluble fibrous protein aggregate that is deposited in the body. Amyloid deposition may be organ-specific (e.g. central nervous system, pancreas, etc.) or systemic. In accordance with this aspect of the invention, amyloidogenic proteins subject to deposition include beta protein precursor, prion, [alpha]-synuclein, tau, ABri precursor protein, ADan precursor protein, amylin, apolipoprotein AI, apolipoprotein All, lyzozyme, cystatin C, gelsolin, protein, atrial natriuretic factor, calcitonin, keratoepithelin, lactoferrin, immunoglobulin light chains, transthyretin, A amyloidosis, [beta]2 - microglobulin, immunoglobulin heavy chains, fibrinogen alpha chains, prolactin, keratin, and medin. Amyloid deposition may occur as its own entity or as a result of another illness (e.g. multiple myeloma, chronic infection, or chronic inflammatory disease).

Therefore, the methods of the present invention can further be used to treat a subject having a condition or disease that is associated with, or resulting from, the deposition of amyloidogenic proteins. Such conditions include, but are not limited to, Alzheimer's disease, diffuse Lewy body disease, Down syndrome, hereditary cerebral hemorrhage with amyloidosis, Creutzfeldt- Jakob disease, Gerstmann-Straussler-Scheinker disease, fatal familial insomnia, British familial dementia, Danish familial dementia, familial corneal amyloidosis, Familial corneal dystrophies, medullary thyroid carcinoma, insulinoma, type 2 diabetes, isolated atrial amyloidosis, pituitary amyloidosis, aortic amyloidosis, plasma cell disorders, familial amyloidosis, senile cardiac amyloidosis, inflammation-associated amyloidosis, familial Mediterranean fever, dialysis- associated amyloidosis, systemic amyloidosis, and familial systemic amyloidosis.

Treatment or prevention of Alzheimer's disease is a preferred feature of the invention.

The invention relates to method of preventing or treating disease in a subject. In one aspect the subject for prevention or treatment may have already been diagnosed with symptoms of a disease characterised by amyloid deposition. In one aspect the subject for treatment has not already been diagnosed with symptoms of a disease characterised by amyloid deposition.

In one aspect the present invention relates to an effect on the deposits of amyloid protein, and in another aspect to an effect on behaviours that are associated with disease states, and in particular prevention or reduction of behaviours associated with Alzheimer's disease.

In one aspect the methods and compositions of the invention have an effect both on amyloid protein deposition and behaviour associated with disease, such as behaviour associated with Alzheimer's disease, although in another aspect the methods and compositions of the invention have an effect either at the level of amyloid deposits or at the level of behaviour.

In one aspect the prevention or reduction in severity of Alzheimer's disease comprises prevention or reduction of loss of memory. In a further aspect the invention relates to relates to improvement in memory. The memory may be spatial memory.

In one further aspect the invention relates to use of compositions as disclosed herein for improved phagocytosis of Amyloid beta, and compositions for use in improved phagocytosis of Amyloid beta.

Without wishing to be bound by theory, the use of a TLR4 agonist free of endotoxin such as an aminoalkyl glucosaminide phosphate, 3D-MPL or MPL, is thought to contribute to the invention by stimulation of the innate immune system.

Thus in one aspect the invention relates to a method of preventing and reducing amyloid deposition or Alzheimer's disease in a subject comprising: selecting a subject with amyloid deposits and stimulating the innate immune system of the selected subject using a TLR4 agonist free of endotoxin under conditions effective to reduce the amyloid deposits.

In one aspect the invention relates to use of compositions as disclosed herein for stimulation of microglial cell activity.

In another aspect microglial cells may be activated by a TLR4 agonist free of endotoxin, or other suitable activator, in culture, before being delivered to the brain for Amyloid β clearance□.

In additional aspects the invention relates to:
A TLR4 agonist free of a TLR2 agonist for use in the methods of the invention disclosed herein, namely for use in the prevention or treatment of Alzheimer's disease and/or the reduction of B amyloid, and in particular in the use in prevention or treatment of adverse behaviours associated with Alzheimer's disease, such as prevention or reduction of loss of memory, or improvement of memory. The memory may be spatial memory. TLR4 agonists may be any of those as described herein.
A TLR4 agonist for use in the prevention or treatment of adverse behaviours associated with Alzheimer's disease, such as prevention or reduction of loss of memory, and/or improvement of memory. The memory may be spatial memory. TLR4 agonists may be those as described herein.

Suitably the agonists of the invention results in an improvement in results in any of the animal assays carried out herein, such as T water maze, nesting or passive avoidance tests, when compared to a suitable control.

In one aspect the present invention utilizes a TLR4 agonist free of endotoxin, for example a pharmaceutical composition comprising an aminoalkyl glucosaminide phosphate (AGP), 3D-MPL or MPL.

TLR4 agonists can be synthesised free from endotoxin or purified free of endotoxin. In one aspect reference to TLR4 agonists 'free of endotoxin' may be a TLR4 agonist containing composition in which endotoxin present has been wholly or partially inactivated or removed in some way, and is thus essentially free of endotoxin activity. In one aspect a composition comprising a TLR4 agonist free of endotoxin is a composition in which the endotoxin level is below maximum acceptable regulatory limits. In one aspect free of endotoxin means that the composition is substantially free of LPS. In one aspect a composition free of endotoxin is one which does not cause a fever when administered.

There is generally no one defined limit for endotoxin, but pharmaceutical limits are generally a maximum of 0.2- 5 EU/kg product, where the FDA has initially defined the Endotoxin Unit (EU) as the endotoxin activity of 0.2 ng of Reference Endotoxin Standard, EC-2 or 5 EU/ng.

In one aspect endotoxin may be detected by the LAL test. Another acceptable approach is the rabbit pyrogen test, which may be used for 3D MPL or AGPs, for example, and in which a solution of 3D MPL or AGP is injected iv into rabbits and rise in temperature is monitored.

Depyrogenation may be achieved by well known techniques including ion exchange chromatography or ultrafiltration.

Suitable TLR4 agonists include MPL and 3D-MPL, which are less toxic than Lipid A. Both are TLR4 agonists. U.S. Patent No. 4,436,727 discloses monophosphoryl lipid A [MPL] and its manufacture. U.S. Patent No. 4,912,094 and re-examination certificate B1 4,912,094 discloses 3-O-deacylated monophosphoryl lipid A [3D MPL] and a method for its manufacture, both of which are incorporated herein by reference.

In one aspect the invention utilises a synthetic TLR4 agonist free of endotoxin. For synthetic TLR4 agonists the endotoxin level may be zero.

In one aspect the synthetic TLR4 agonist may be a synthetic disaccharide molecules, similar in structure to MPL and 3D-MPL or may be synthetic monosaccharide molecules, such as the aminoalkyl glucosaminide phosphate compounds disclosed in, for example, WO9850399, WO0134617, WO0212258, WO3065806, WO04062599, WO06016997, WO0612425, WO03066065, and WO0190129 the disclosure of which is herein incorporated by reference. Such molecules have also been described in the scientific and patent literature as lipid A mimetics, which also form an aspect of the present invention.

The TLR4 agonist may be a lipid A mimetic. Lipid A mimetics suitably share some functional and/or structural activity with lipid A, and in one aspect are recognised by TLR4 receptors. AGPs as described herein are sometimes referred to as lipid A mimetics in the art. Lipid A mimetics in one aspect are less toxic than lipid A.

In one aspect the aminoalkyl glucosaminide phosphate (AGP) is one in which an aminoalkyl (aglycon) group is glycosidically linked to a 2-deoxy-2-amino-a-D- glucopyranose (glucosaminide) to form the basic structure of the claimed molecules. The compounds are phosphorylated at the 4 or 6 carbon on the glucosaminide ring. Further, the compounds possess three 3-alkanoyloxyalkanoyl residues comprising a primary and secondary fatty acyl chain, each carbon chain consisting of from 2-24 carbon atoms, and preferably from 7-16 carbon atoms. In one preferred aspect, each primary chain contains 14 carbon atoms and each secondary chain has between 10 and 14 carbon atoms.

In one aspect the AGP compounds are described by the general formula:

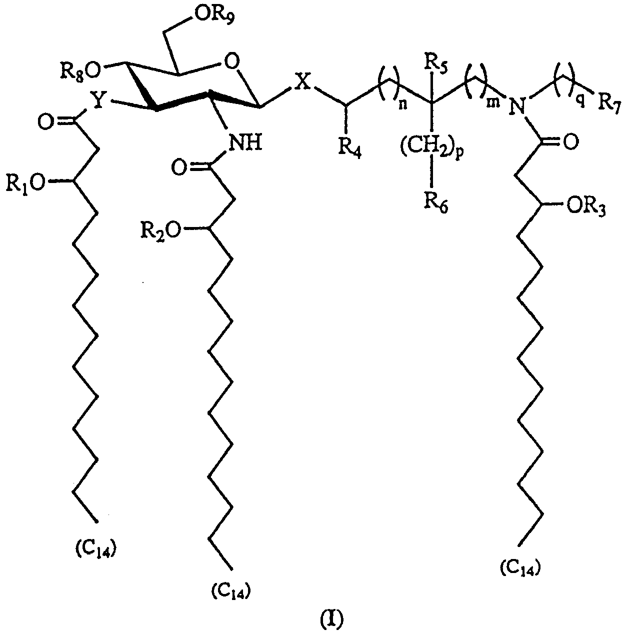

Such compounds comprise a 2-deoxy-2-amino-a-D-glucopyranose (glucosamine) in glycosidic linkage with an aminoalkyl (aglycon) group. Compounds are phosphorylated at the 4 or 6 carbon on the glucosamine ring and have three alkanoyloxyalkanoyl residues. The compounds are described generally by Formula I, wherein X represents an oxygen or sulfur atom, Y represents an oxygen atom or NH group, "n", "m", "p" and "q" are integers from 0 to 6, R1, R2, and R3 represent normal fatty acyl residues having 7 to 16 carbon atoms, R4 and R5 are hydrogen or methyl, R6 and R7 are hydrogen, hydroxy, alkoxy, phosphono, phosphonooxy, sulfo, sulfooxy, amino, mercapto, cyano, nitro, formyl or carboxy and esters and amides thereof; R8 and R9 are phosphono or hydrogen. The configuration of the 3' stereogenic centers to which the normal fatty acyl residues are attached is R or S, but preferably R. The stereochemistry of the carbon atoms to which R4 or R5 are attached can be R or S. All stereoisomers, both enantiomers and diastereomers, and mixtures thereof, are considered to fall within the scope of the subject invention.

The heteroatom X of such compounds of the subject invention can be oxygen or sulfur. In a preferred embodiment, X is oxygen. Although the stability of the molecules could be effected by a substitution at X, the immunomodulating activity of molecules with these substitutions is not expected to change.

The number of carbon atoms between heteroatom X and the aglycon nitrogen atom is determined by variables "n" and "m". Variables "n" and "m" can be integers from 0 to 6. In a preferred embodiment, the total number of carbon atoms between heteroatom X and the aglycon nitrogen atom is from about 2 to about 6 and most preferably from about 2 to about 4.

Such compounds are aminoalkyl glucosamine compounds which are phosphorylated. Compounds can be phosphorylated at position 4 or 6 (R8 or R9) on the glucosamine ring and are most effective if phosphorylated on at least one of these positions. In a preferred embodiment, R8 is phosphono and R9 is hydrogen.

Such compounds are hexaacylated, that is they contain a total of six fatty acid residues. The aminoalkyl glucosamine moiety is acylated at the 2- amino and 3-hydroxyl groups of the glucosamine unit and at the amino group of the aglycon unit with 3-hydroxyalkanoyl residues. In Formula I, these three positions are acylated with 3-hydroxytetradecanoyl moieties. The 3-hydroxytetradecanoyl residues are, in turn, substituted with normal fatty acids (R1-R3), providing three 3-n- alkanoyloxytetradecanoyl residues or six fatty acid groups in total.

The chain length of normal fatty acids R1-R3 can be from about 7 to about 16 carbons. Preferably, R1-R3 are from about 9 to about 14 carbons. The chain lengths of these normal fatty acids can be the same or different. Although, only normal fatty acids are described, it is expected that unsaturated fatty acids (i.e. fatty acid moieties having double or triple bonds) substituted at R1,-R3 on the compounds would produce biologically active molecules. Further, slight modifications in the chain length of the 3-hydroxyalkanoyl residues are not expected to dramatically effect biological activity.

Specific examples of AGP's include: CRX- 527 which is disclosed in JBC 2004 279, No 6, page 4440-4449 (http://www.jbc.org/content/279/6/4440.full.pdf).

WO0212258 and WO3065806 disclose additional embodiments of AGPs having a cyclic aminoalkyl (aglycon) linked to a 2-deoxy-2-amino-a-D- glucopyranose (glucosaminide), commonly referred to as "cyclic AGP's."

Reference generally to AGPs herein includes both cyclic and non cyclic AGPs.

Cyclic AGPs possess three 3-alkanoyloxyalkanoyl residues comprising a primary and secondary fatty acyl chain, each carbon chain consisting of from 2-24 carbon atoms, and preferably from 7-16 carbon atoms. In one preferred aspect each primary chain contains 14 carbon atoms and each secondary carbon chain has between 10 and 14 carbon atoms per chain.

The cyclic AGPs are described by the general formula II:

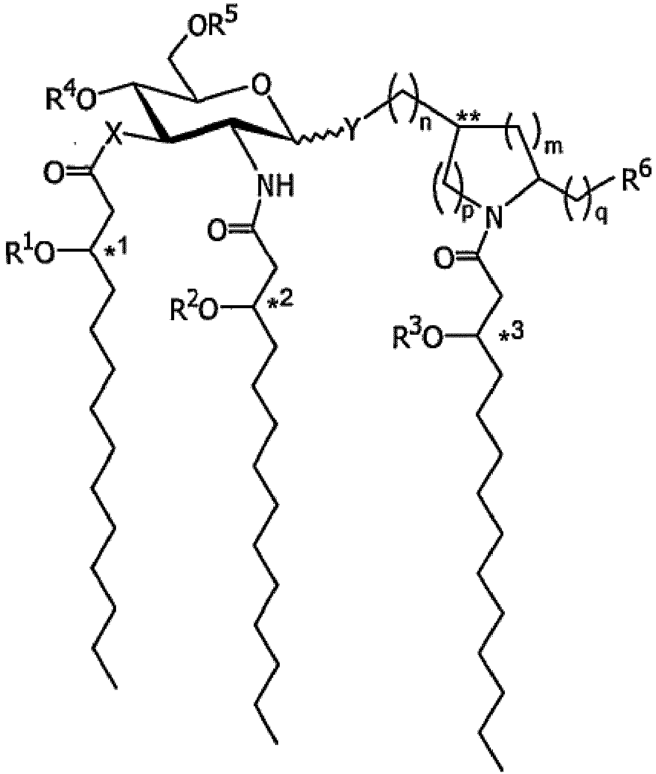

These compounds comprise a 2-deoxy-2-amino-p-D-glucopyranose (glucosamine) glycosidically linked to an cyclic aminoalkyl (aglycon) group. The compounds are phosphorylated at the 4 or 6-position of the glucosamine ring and acylated with alkanoyloxytetradecanoyl residues on the aglycon nitrogen and the 2 and 3-positions of the glucosamine ring. The compounds are described generally by formula (II): and pharmaceutically acceptable salts thereof, wherein X is-O-or NH-and Y is-O-or-S-; R1, R2, and R3 are each independently a (C2-C24) acyl group, including saturated, unsaturated and branched acyl groups; R4 is -H or -PO₃R7R8, wherein R7 and R8 are each independently H or (C1-C4) alkyl ; R5 is -H, -CH₃ or-PO₃R9R10, wherein R9 and RI0 are each independently selected from-H and (CI-C4) alkyl ; R6 is independently selected from H, OH, (CI-C4) alkoxy, -PO₃R11R12, -OPO₃R11R12, -SO₃R11, -OSO₃R11,-NR11R12, -SR11, -CN, -NO₂, -CHO, -CO₂R11, and -CONR11R12, wherein R11 and R12 are each independently selected from H and (CI-C4) alkyl; with the proviso that when R4 is- Po₃R7R8, R5 is other than-P0 R9R10, wherein"*1-3"and"**"represent chiral centers; wherein the subscripts n, m, p and q are each independently an integer from 0 to 6, with the proviso that the sum of p and m is from 0 to 6.

In some embodiments, the compounds of the present invention contain an- -O-at X and Y, R4 is PO₃R7R8, R5 and R6 are H, and the subscripts n, m, p, and q are integers from 0 to 3. In a more preferred embodiment, R7 and R8 are -H. In an even more preferred embodiment, subscript n is 1, subscript m is 2, and subscripts p and q are 0. In yet an even more preferred embodiment, R1, R2, and R3 are tetradecanoyl residues. In a still more preferred embodiment, *1-3 are in the R configuration, Y is in the equatorial position, and ** is in the S configuration (N-[(R)-3-tetradecanoyloxytetradecanoyl]-(S)-2-pyrrolidinomethyl 2-deoxy-4-0-phosphono-2-[(R)-3- tetradecanoyloxytetradecanoylamino]-3-0-[(R)-3-tetradecanoyloxytetradecanoyl]-p-D- glucopyranoside and pharmaceutically acceptable salts thereof

### Preferred cyclic structures include:

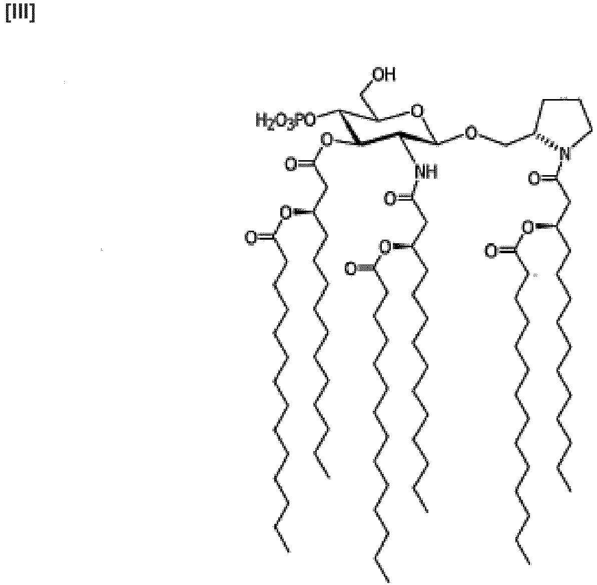

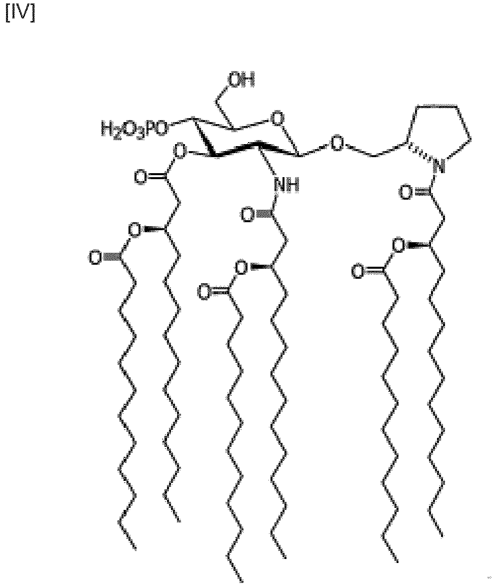

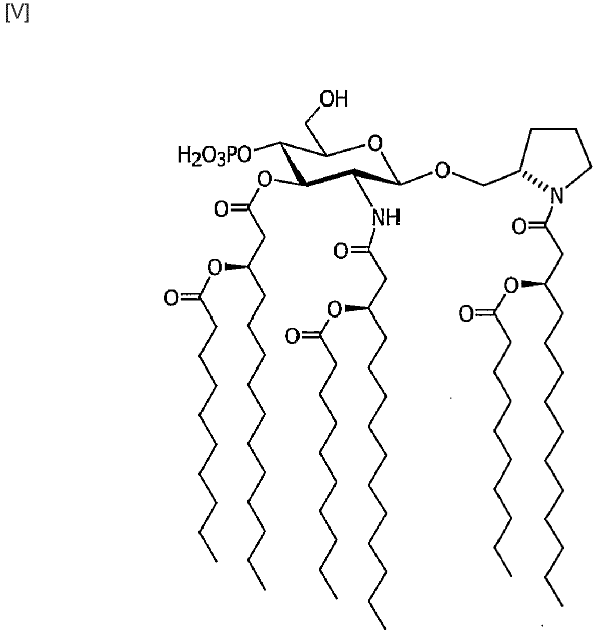

Formula V is CRX 590.

In another aspect the TLR4 receptor ligand is an AGP having one or more ether linked rather than ester linked primary and/or secondary lipid groups. In this embodiment, R1-R3 represent straight chain alkyl groups and not acyl groups, making the groups R1O-, R2O-, and R3O- alkoxy rather than alkanoyloxy groups and the attachment to the primary acyl chain an ether rather than an ester linkage. In the case of an ether-linked primary lipid group, the 3-alkanoyloxyalkanoyl residue attached to the 3-hydroxy group of the glucosamine unit is replaced with either a 3-alkanoyloxyalkyl moiety or a 3-alkoxyalkyl moiety, making the attachment of the primary lipid group to the glucosamine 3-position an ether rather than an ester linkage.

A general formula for ethers is that of formula IV of WO2006016997.

An example of a preferred compound is CRX601.

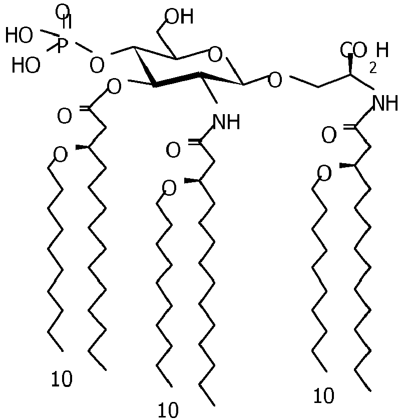

In another aspect, the AGP molecule may have different number of carbons in the molecule's primary chains and/or secondary chains. Such compounds are disclosed in WO04062599 and WO06016997. As with other AGPs, each carbon chain may consist of from 2-24 carbon atoms, and preferably from 7-16 carbon atoms. In one preferred aspect each primary chain contains 14 carbon atoms and each secondary carbon chain has between 10 and 14 carbon atoms per chain.

Such compounds are represented by the following structures:

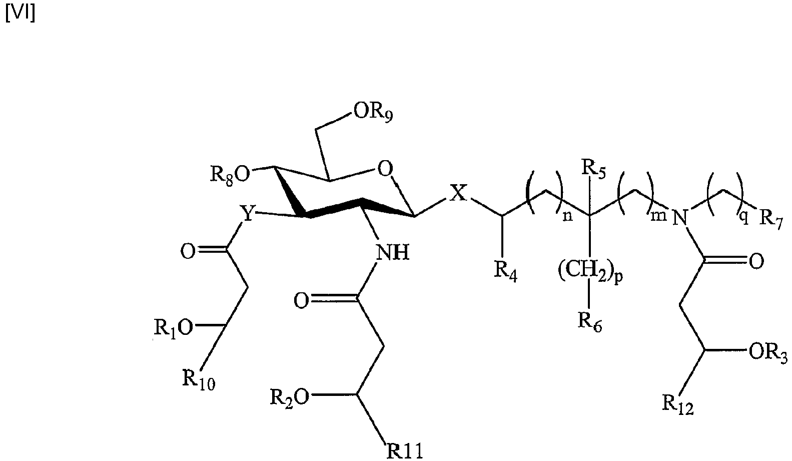

wherein X is selected from the group consisting of O and S at the axial or equatorial position; Y is selected from the group consisting of O and NH; n, m, p and q are integers from 0 to 6; R1, R2 and R3 are the same or different and are fatty acyl residues having from 1 to about 20 carbon atoms and where one of R1, R2 or R3 is optionally hydrogen; R4 and R5 are the same or different and are selected from the group consisting of H and methyl; R6 and R7 are the same or different and are selected from the group consisting of H, hydroxy, alkoxy, phosphono, phosphonooxy, sulfo, sulfooxy, amino, mercapto, cyano, nitro, formyl and carboxy, and esters and amides thereof; R8 and R9 are the same or different and are selected from the group consisting of phosphono and H, and at least one of R8 and R9 is phosphono; R10, R11 and R12 are independently selected from straight chain unsubstituted saturated aliphatic groups having from 1 to 10 carbon atoms; or a pharmaceutically acceptable salt thereof. 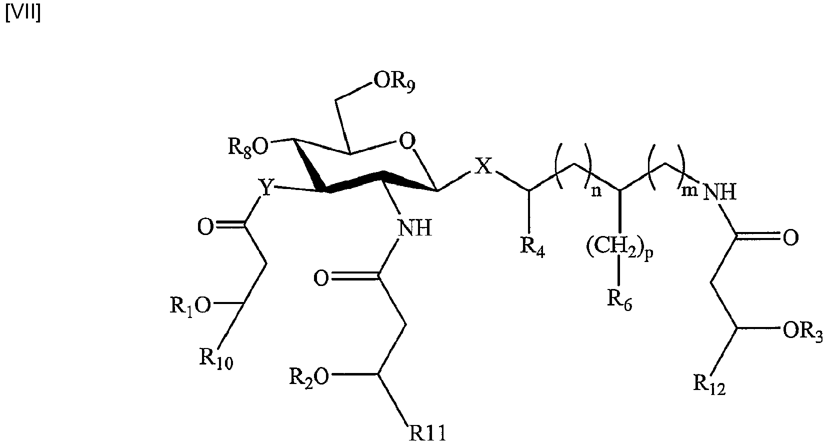 wherein X is selected from the group consisting of O and S at the axial or equatorial position; Y is selected from the group consisting of O and NH ; n and m are 0; R1, R2 and R3 are the same or different and are fatty acyl residues having from 1 to about 20 carbon atoms and where one of R1, R2 or R3 is optionally hydrogen; R4 is selected from the group consisting of H and methyl; p is 1 and R6 is COOH or p is 2 and R6 is OP0₃H₂ ; R8 and R9 are the same or different and are selected from the group consisting of phosphono and H, and at least one of R8 and R9 is phosphono; and R10, R11 and R12 are independently selected from straight chain unsubstituted saturated aliphatic groups having from 1 to 10 carbon atoms; or a pharmaceutically acceptable salt thereof. 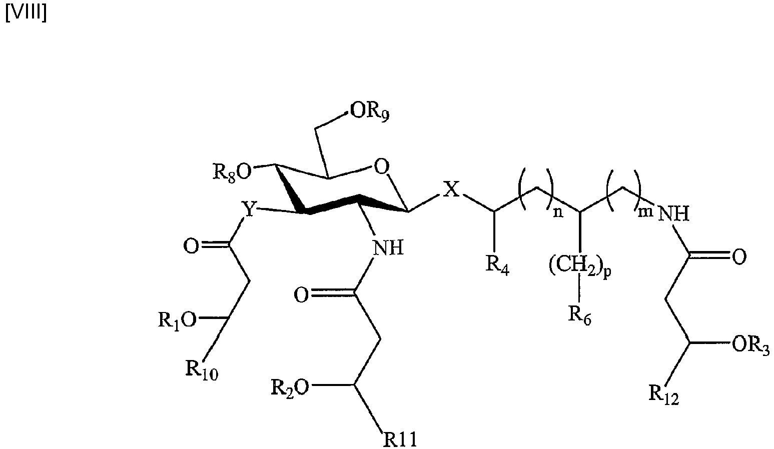 wherein X is selected from the group consisting of O and S at the axial or equatorial position; Y is selected from the group consisting of O and NH; n, m, p and q are integers from 0 to 6; R1, R2 and R3 are the same or different and are straight chain saturated aliphatic groups (i.e., straight chain alkyl groups) having from 1 to about 20 carbon atoms and where one of R1, R2 or R3 is optionally hydrogen; R4 and R5 are the same or different and are selected from the group consisting of H and methyl; R6 and R7 are the same or different and are selected from the group consisting of H, hydroxy, alkoxy, phosphono, phosphonooxy, sulfo, sulfooxy, amino, mercapto, cyano, nitro, formyl and carboxy, and esters and amides thereof; R8 and R9 are the same or different and are selected from the group consisting of phosphono and H, and at least one of R8 and R9 is phosphono; R10, R11 and R12 are independently selected from straight chain unsubstituted saturated aliphatic groups having from 1 to 11 carbon atoms;
or a pharmaceutically acceptable salt thereof.

The general formula may also comprise an R5 group, at the same position as shown in formula VI above, wherein R5 is selected from the group consisting of H and methyl.

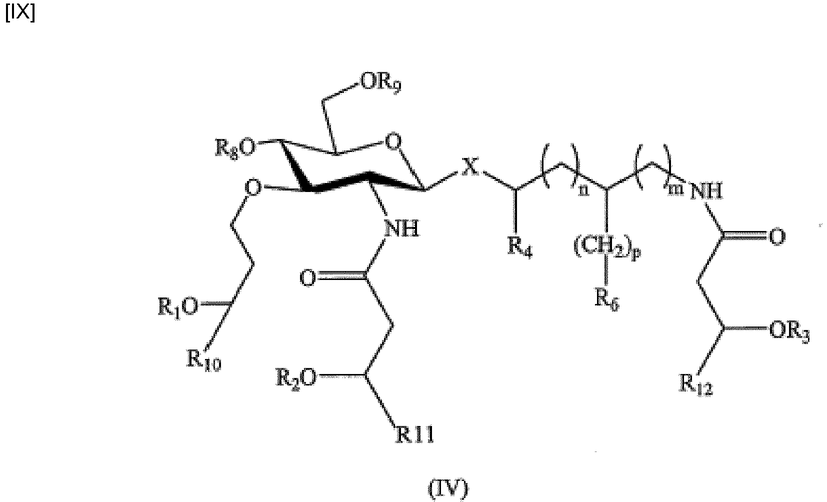

Yet another type of compound of this invention has the formula (IV): wherein Y is now fixed as oxygen; X is selected from the group consisting of O and S at the axial or equatorial position; n and m are 0; R1, R2 and R3 are the same or different and are fatty acyl residues having from 1 to about 20 carbon atoms and where one of R1, R2 or R3 is optionally hydrogen; R4 is selected from the group consisting of H and methyl; p is 0 or1 and R6 is COOH, or p is 1 or 2 and R6 is OPO3H2 ; R8 and R9 are the same or different and are selected from the group consisting of phosphono and H, and at least one of R8 and R9 is phosphono; and R10, R11 and R12 are independently selected from straight chain unsubstituted saturated aliphatic groups having from 1 to 10 carbon atoms; or a pharmaceutically acceptable salt thereof.

These compounds thus have two acylated chains and one non-acylated ether chain.

Processes for making AGPs are also disclosed in WO0612425.

Methods for preventing and treating diseases by administering AGPs in the absence of exogenous antigens are disclosed in WO03066065 and WO0190129.

Other AGP structures such as CRX 524 are disclosed in Infection and Immunity, May 2005, p. 3044-3052 Vol. 73, No. 5.

The present invention further relates to pharmaceutical compositions comprising a TLR4 agonist free of endotoxin, such as an aminoalkyl glucosaminide phosphate (AGP), 3D-MPL or MPL.

Compositions may alternatively consist, or consist essentially of, a TLR4 agonist free of endotoxin, such as MPL, 3D MPL and AGPs, in that these agents may be respectively delivered alone or in combination with excipients such as carriers, excipients, buffers and the like.

In further aspects the TLR4 agonist free of endotoxin may be formulated with other components which may enhance efficacy.

The TLR4 agonist free of endotoxin may be combined with other pharmaceutically active agents.

In one aspect a pharmaceutical composition comprising 3D-MPL consists, or consists essentially of, 3D MPL in combination with a saponin, such as QS21, and liposomes. In one aspect the composition consists or consists essentially of AS01 B (see for example EP822831). In one aspect treatment with 3DMPL or use of 3DMPL according to any of the previous aspects of the invention includes the use of AS01B.

In one aspect a pharmaceutical composition comprising an AGP consists or consists essentially of an AGP in combination with an oil in water emulsion, such as AS03 (for example, as disclosed in EP868918). In one aspect treatment with an AGP or use of an AGP according to any of the previous aspects of the invention includes the use of an AGP in combination with an oil in water emulsion.

In one aspect the composition of the invention does not comprise an amyloid polypeptide or fragment thereof. In one aspect it does not comprise or an Alzheimer's disease specific antigen, such as Amyloid beta or fragment thereof. It may comprise an antigen or agent specific for a disease which is not characterised by amyloid deposition.

Thus in one aspect the invention relates to a pharmaceutical composition comprising a TLR4 agonist free of endotoxin such as an aminoalkyl glucosaminide phosphate, MPL or 3D MPL and pharmaceutically acceptable excipient in the absence of a disease specific antigen, such as Alzheimer's antigen.

In one aspect the invention relates to pharmaceutical compositions, and use of compositions, which consist of, or consist essentially of,
an AGP;
an AGP in combination with an oil in water emulsion;
3D-MPL;
3D-MPL in combination with QS21 and liposomes.

In one aspect the invention relates to pharmaceutical compositions, and use of compositions, which consist of, or consist essentially of,
an AGP;
an AGP in combination with an oil in water emulsion;
3D-MPL; and
3D-MPL in combination with QS21 and liposomes;

in combination with an excipient, suitable for use in an individual such as a human.

Generally a pharmaceutical composition is one which is suitable for delivery to a human.

In one aspect the TLR4 agonist free of endotoxin, such as aminoalkyl glucosaminide phosphate, 3D MPL or MPL is in the form of a pharmaceutical composition formulated with an immunostimulant. The immunostimulant may be a saponin, such as QS21, or may be an oil in water emulsion, such as an emulsion additionally comprising tocopherol, or may be any other suitable immunostimulant.

In one aspect the TLR4 agonist free of endotoxin is AS03 in combination with CRX601

In one aspect the immunostimulant is a stimulator of the innate immune response, and in one aspect is not an antigen associated with a specific disease.

In one aspect the TLR4 agonist free of endotoxin is not formulated with a CpG oligodeoxynucleotide.

In one aspect a composition for use in the invention comprises a TLR4 agonist free of endotoxin, combined with an oil in water emulsion containing squalene, alpha tocopherol and polysorbate 80, for example having a human dose of 10.69 mg squalene, 11.86 mg DL-α-tocopherol , 4.86 mg polysorbate 80, or components in that general ratio.

In one aspect the composition for use in the invention comprises a combination of a TLR4 agonist free of endotoxin such, as monophosphoryl lipid A, and a saponin derivative, particularly the combination of QS21 and 3D-MPL as disclosed in WO 94/00153, or a less reactogenic composition where the QS21 is quenched with cholesterol as disclosed in WO96/33739. A particularly potent adjuvant formulation involving QS21 3D-MPL and tocopherol in an oil in water emulsion is described in WO95/17210 and is a suitable formulation.

The present invention also relates to compositions as disclosed herein in these scientific results.

As mentioned above, the compositions of the present invention can be used in one or more of preventing or reducing effect on deposits of amyloid protein, stimulation of innate immunity via microglia cells, increasing amyloid phagocytosis and preventing or reducing behaviours that are associated with disease states such as Alzheimer's disease. The examples provided herein give suitable methods for assessing of these parameters.

Beta amyloid deposits may be measured as a function of the area of plaques in a brain section, or assessed by total protein concentration, as described in the attached Examples. Other suitable methods are disclosed in WO2009105641, incorporated herein by reference.

Effects of the treatments and compositions of the invention on behaviour associated with Alzheimer's disease may be assessed in human patients, or in animal models, for example. Suitable animal models include the mouse APP model for Alzheimer's disease, the PS1 mouse model and the APP/PS1 model. See Richard, K.L. et al. J Neurosci 28, 5784-5793 (2008).

Suitable animal (rodent) tests include one or more of the T-water maze test, Passive avoidance test, or nesting behaviour tests as described herein (Filali M, et al Cognitive and non-cognitive behaviours in an APPswe/PS1 bigenic model of Alzheimer's disease. Genes Brain Behav. 2009 Mar;8(2):143-8. Epub 2008 Dec 3. PubMed PMID: 19077180.). Other behavioural tests that may be employed are described in WO2009105641, incorporated herein by reference.

Stimulation of the innate immune system may be effected by, and/or measured by, stimulation of microglia. In another aspect the innate immune response may be assessed by the triggering transcriptional activation of TLR2 in brain tissues, for example in appropriate animal mouse models.

The preparations of the present invention may be used to protect or treat a mammal by means of administering via systemic or mucosal route. These administrations may include injection via the intramuscular, intraperitoneal, intradermal or subcutaneous routes; or via mucosal administration to the oral/alimentary, respiratory, genitourinary tracts. The composition of the invention may be administered as a single dose, or multiple doses. In addition, the compositions of the invention may be administered by different routes for priming and boosting, for example, IM priming doses and IN for booster doses.

The composition of the present invention may be administered alone or with suitable pharmaceutical carriers, and can be in solid or liquid form, such as tablets, capsules, powders, solutions, suspensions, or emulsions. An aminoalkyl glucosaminide phosphate (AGP), 3D MPL or MPL or any composition of the invention, may be formulated into a "vaccine," and administered in free solution, or formulated with an adjuvant, or excipient. Vaccine preparation is generally described in Vaccine Design ("The subunit and adjuvant approach" (eds Powell M. F. & Newman M.J.) (1995) Plenum Press New York). Encapsulation within liposomes is described by Fullerton, US Patent 4,235,877. The vaccines of the present invention may be stored in solution or lyophilized.

Effective doses of the compositions of the present invention, for the treatment of a subject having amyloid deposits or AD vary depending upon many different factors, including means of administration, target site, physiological state of the patient, other medications administered, physical state of the patient relative to other medical complications, and whether treatment is prophylactic or therapeutic. Treatment dosages need to be titrated to optimize safety and efficacy. The amount of agonist depends on whether an adjuvant is also administered. Subject doses of the agonist described herein typically range from about 0. 1µg to 50mg per administration, which depending on the application could be given daily, weekly, or monthly and any other amount of time therebetween. More typically mucosal or local doses range from about 10µg to 10mg per administration, and optionally from about 100µg to 1 mg, with 2-4 administrations being spaced days or weeks apart. More typically, immune stimulant doses range from 1µg to 10 mg per administration, and most typically 10µg to 1 mg, with daily or weekly administrations. Doses of the compounds described herein for parenteral delivery e.g., for inducing an innate immune response, or in specialized delivery vehicles typically range from about 0.1 µg to 10 mg per administration, which depending on the application could be given daily, weekly, or monthly and any other amount of time therebetween. More typically parenteral doses for these purposes range from about 10µg to 5mg per administration, and most typically from about 100µg to 1 mg, with 2-4 administrations being spaced days or weeks apart. In some embodiments, however, parenteral doses for these purposes may be used in a range of 5 to 10,000 times higher than the typical doses described above.

The teaching of all references in the present application, including patent applications and granted patents, are herein fully incorporated by reference. Any patent application to which this application claims priority is incorporated by reference herein in its entirety in the manner described herein for publications and references.

Any aspect or feature of the invention may be combinable with any other aspect or feature of the invention, even where disclosed in a specific example, except where obvious from the context.

For the avoidance of doubt the terms 'comprising', 'comprise' and 'comprises' herein is intended by the inventors to be optionally substitutable with the terms 'consisting of', 'consist of', and 'consists of', respectively, in every instance. As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps.

Embodiments herein relating to "vaccine compositions" of the disclosure are also applicable to embodiments relating to "immunogenic compositions" of the disclosure, and vice versa. The term "about" (or "around") in all numerical values allows for a 5% variation, i.e. a value of about 1.25% would mean from between 1.19%-1.31%.

The use of the word "a" or "an" when used in conjunction with the term "comprising" in the claims and/or the specification may mean "one," but it is also consistent with the meaning of "one or more," "at least one," and "one or more than one." The use of the term "or" in the claims is used to mean "and/or" unless explicitly indicated to refer to alternatives only or the alternatives are mutually exclusive, although the disclosure supports a definition that refers to only alternatives and "and/or." Throughout this application, the term "about" is used to indicate that a value includes the inherent variation of error for the measurement, the method being employed to determine the value, or the variation that exists among the study subjects.

The term "or combinations thereof" as used herein refers to all permutations and combinations of the listed items preceding the term. For example, "A, B, C, or combinations thereof is intended to include at least one of: A, B, C, AB, AC, BC, or ABC, and if order is important in a particular context, also BA, CA, CB, CBA, BCA, ACB, BAC, or CAB. Continuing with this example, expressly included are combinations that contain repeats of one or more item or term, such as BB, AAA, BBC, AAABCCCC, CBBAAA, CABABB, and so forth. The skilled artisan will understand that typically there is no limit on the number of items or terms in any combination, unless otherwise apparent from the context.

All of the compositions and/or methods disclosed and claimed herein can be made and executed without undue experimentation in light of the present disclosure. While the compositions and methods of this disclosure have been described in terms of suitable embodiments, it will be apparent to those of skill in the art that variations may be applied to the compositions and/or methods and in the steps or in the sequence of steps of the method described herein without departing from the concept, spirit and scope of the disclosure. All such similar substitutes and modifications apparent to those skilled in the art are deemed to be within the spirit, scope and concept of the disclosure as defined by the appended claims.

It will be understood that particular embodiments described herein are shown by way of illustration and not as limitations of the disclosure. The principal features of this disclosure can be employed in various embodiments without departing from the scope of the disclosure. Those skilled in the art will recognize, or be able to ascertain using no more than routine study, numerous equivalents to the specific procedures described herein. Such equivalents are considered to be within the scope of this disclosure and are covered by the claims. All publications and patent applications mentioned in the specification are indicative of the level of skill of those skilled in the art to which this disclosure pertains. All publications and patent applications are herein incorporated by reference to the same extent as if each individual publication or patent application was specifically and individually indicated to be incorporated by reference.

The disclosure will be further described by reference to the following, non-limiting, examples:

### Examples

In the examples and Figures described below, all reference to MPL are references to 3D-MPL.

### Example 1

Innate activation into the brain after a single injection of CRX524 or CRX527, or CRX601 or 3D MPL.

TLR4 ligands such as LPS have been shown to induce a concomitant expression of TLR2 mRNA (Fan, J., Randall, S.F., Malik, AB., 2003. TLR4 signaling induces TLR2 expression in endothelial cells via neutrophil NADPH oxidase. 112 (8): 1234. J Clin Invest.*).* Until now, no other LPS mimetic as been shown in the literature to induce such innate activation within the brain from peripheral injection. One of the first immediate receptor within the brain activated by TLR4 agonist is the TLR2 mRNA (P. A. Carpentier, D. S. Duncan, and S. D. Miller, "Glial toll-like receptor signaling in central nervous system infection and autoimmunity," Brain, Behavior, and Immunity, vol. 22, no. 2, pp. 140-147, 2008.).

In order to evaluate the innate activation within the brain, we have performed in situ hybridization 24 hr following the systemic injection of either 3D MPL, CRX524 or CRX527 or CRX601 using the intra peritoneal route.

Specifically GSK Bio injected different compositions in C57BL/6J mice for evaluating the potency of those adjuvants after 24hrs of peripheral injection to trigger microglial cell activation in the brain. Animal study groups were:
- 1.: NaCl 0.9 % (n=5), ip injections
- 2.: CRX524 (20 µg/mouse, 130 µl, n=5/ group), ip injections
- 3.: CRX527 (20 µg/mouse, 130 µl, n=5/ group), ip injections
- 4.: CRX601 (20 µg/mouse, 130 µl, n=5/ group), ip injections
- 5.: 3D MPL (50 µg/mouse, 130 µl, n=5/ group), ip injections
- 6.: AS01B (1/10th of human dose µg/kg, 50 µl, n=5/ group), im (intra muscular) injections

GSK Bio performed the injections in C57BL6 mice at the Centre de biologie experimentale de l'INRS-IAF under an IACUC approved protocol. Twenty four hours after the injections, mice were deeply anesthetized via an intraperitoneal injection of a mixture of ketamine hydrochloride and xylazine and then perfused intracardially with ice-cold 0.9% saline, followed by 4% paraformaldehyde (PFA) in a 0.1 M borax buffer, pH 9.5, at 4°C. Brains were rapidly removed from skulls, postfixed in PFA 1-3 d at 4°C and sent to the CHUL. They were then cryoprotected in 10% sucrose diluted in PFA overnight. The frozen brains were sectioned into 25-µm-thick coronal sections using a microtome (Reichert-Jung, Cambridge Instruments Company), and slices were collected in a cold cryoprotectant solution (0.05 M sodium phosphate buffer, pH 7.3, 30% ethylene glycol, and 20% glycerol, stored at -20°C).

### In situ hybridization and immunohistochemistry.

Every 12th section of brain slices, starting from the end of the olfactory bulb to the end of the cerebral cortex, was mounted on Colorfrost/Plus microscope slides (Fisher Scientific). *In situ* hybridization histochemical localization of TLR2 transcript was performed using ³⁵S-labeled cRNA probe. Riboprobe synthesis and preparation and *in situ* hybridization were performed according to a protocol described previously (Laflamme et al., 1999 (Neuroscience 1999 Jan;88(1):223-40); Laflamme and Rivest, 2001 (FASEB Journal. 2001;15:155-163) ; Nadeau and Rivest, 2000 (Neurosci 20: 3456-3468); Naert et al., 2009).

Dual labeling combining immunocytochemistry and *in situ* hybridization was performed as described previously (Laflamme and Rivest, 2001; Nadeau and Rivest, 2000 infra) to localize TLR2 transcripts in microglia (iba-1 staining). We used a polyclonal rabbit anti-ionized calcium binding adaptor molecule 1 (iba-1, 1:3000, Wako Chemicals) to stain microglia. All images were captured using a Nikon Eclipse 80i microscope equipped with a digital camera (Qlmaging), processed to enhance contrast and sharpness using Adobe Photoshop 7 (Adobe Systems), and then assembled using Adobe Illustrator (Adobe Systems). The images depicted by the different panels are representative of the signal detected on the slides for each group of mice.

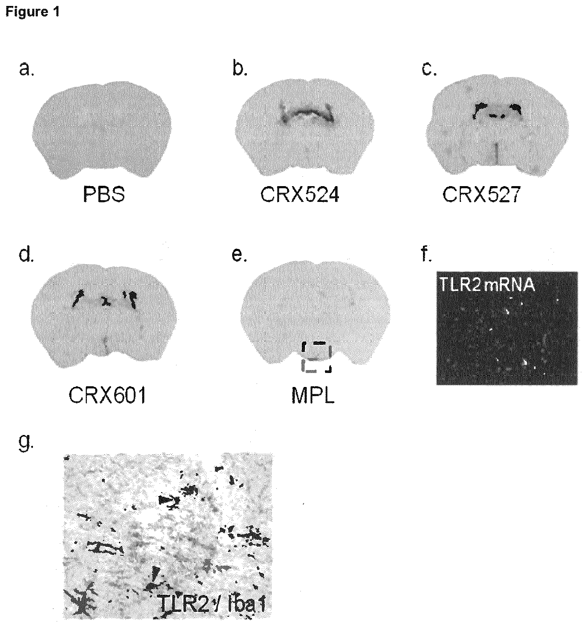

Herein, (Figure 1), we show that TLR2 mRNA is clearly activated in ventricular regions and in the choroid plexus following the injection of CRX524 (panel b), CRX527 (panel c) and CRX601 (panel d). In a lesser extent, we are showing that 3D MPL activates TLR2mRNA transcription (panel e and f). To determine the localization of the TLR2 transcripts, we have performed a counter immunostaining using the microglia cell marker (Iba1) (see panel g).

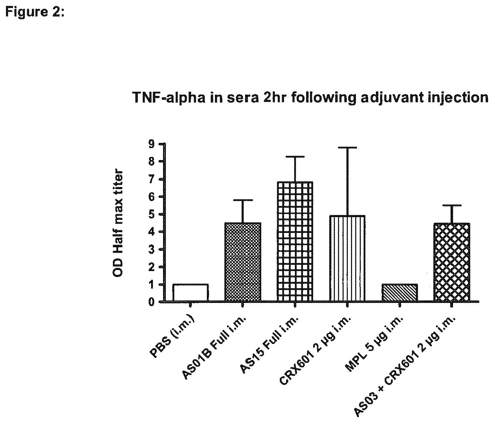

### Example 2, Figure 2

Cytokine (TNFα) was measured in mouse sera following (2hr post injection) peripheral injection of 3D MPL or other TLR4 agonist free of endotoxin molecules. Results are shown in Figure 2.

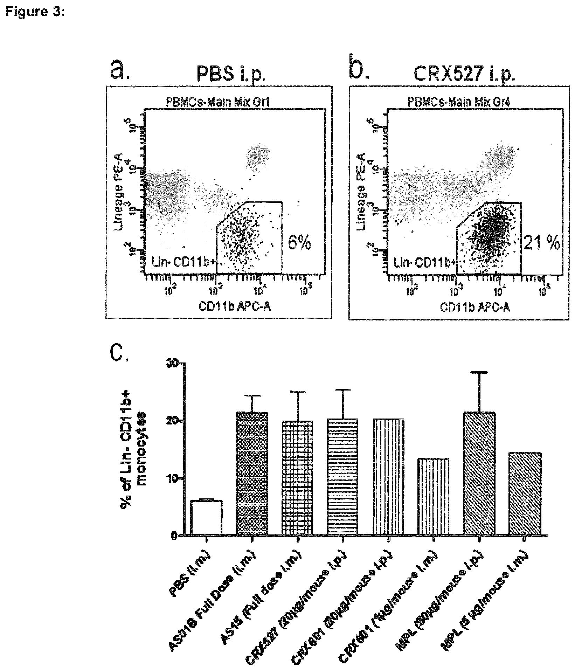

### Example 3, Figures 3 - 7

Upregulation of circulating monocytes numbers following the injection of compositions comprising TLR4 agonists free of endotoxin such as 3D MPL, AS01B, AS15, CRX527 or CRX601.

Monocytes are the peripheral blood precursor cells of microglia (Rezaie, P., et al 1999. Microglia in the human fetal spinal cord-patterns of distribution, morphology and phenotype. Brain Res. Dev. Brain Res. 115:71-81: Mildner et al Nat Neurosci. 2007 Dec;10(12):1544-53. Epub 2007 Nov 18. PubMed PMID: 18026096.). During embryonic development, microglia populate the CNS from myeloid lineage precursors in the bone marrow and these cells are circulating in the peripheral blood before becoming macrophages in their infiltrating tissues, such as microglia are for the brain. Markers such as CD11b and Ly6C are immunologicals markers that are present on peripheral blood monocytes and persist when they are infiltrating the brain (Mildner et al., 2007 infra, Lebson L, et al Trafficking CD11b-positive blood cells deliver therapeutic genes to the brain of amyloid-depositing transgenic mice. J Neurosci. 2010 Jul 21;30(29):9651-8. PubMed PMID: 20660248.). To investigate whether 3D MPL containing adjuvants and s are contributing to induce the number of monocytes in the peripheral blood, we have performed a single injection of those molecules and measure after 24hrs (post injection) the CD11b+ monocytes number by a flow cytometry method (Mildner A, et al Microglia in the adult brain arise from Ly-6ChiCCR2+ monocytes only under defined host conditions. Nat Neurosci. 2007 Dec;10(12):1544-53. Epub 2007 Nov 18. PubMed PMID: 18026096). As described in figure 3, we are showing an increase of CD11b + monocyte number having that phenotypic composition of markers :CD11b+, lineage cocktail negative (CD3-, B220-, NK1.1-,Ly6G-) after the injection of 3D MPL or AS15 or AS01 B or CRX527 or CRX601. Comparing to a normal monocyte number count in the peripheral blood, which is 5% whereas the intramuscular (i.m.) injection of AS01 B, and AS15 are showing an increase of the number of circulating monocytes to up to 20%. Moreover, the injection of 3D MPL or CRX601 or CRX601 using the intraperitoneal route is also showing a 22% of total monocytes in the peripheral blood. In lower extend, the intramuscular injection of CRX601 or 3D MPL have demonstrated an increase of monocytes number (up to 12% and 13% respectively).

### Results

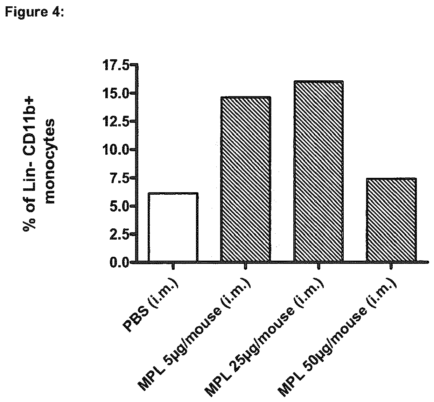

Herein in Figure 4, a 5ug dose of 3D MPL is enough to trigger a double increase of CD11b+ monocyte in the peripheral blood. The higher dose of intramuscular 3D MPL (50ug) is showing a drop of that peripheral blood monocyte count after 24 hours.

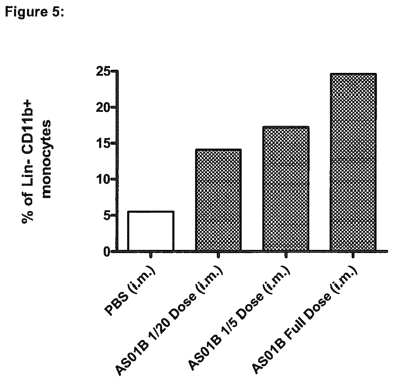

In figure 5 a dilution of 1/20 of AS01 B is enough to trigger an increase of the monocyte count within the peripheral blood. A constant increase is noted until the mouse full dose, i.e. the AS01 B mouse full dose is containing 5 µg of 3D MPL and 5 µg of QS21.

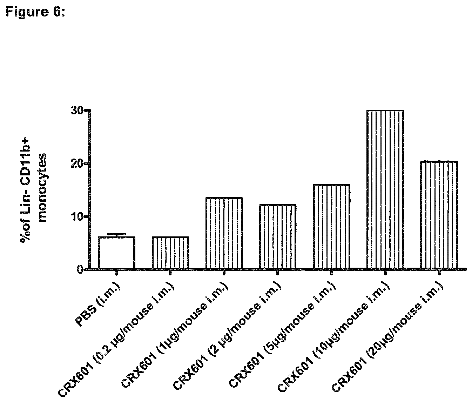

In Figure 6 we have performed a thorough dilution analysis of CRX601 to identify the optimal dose of CRX601 triggering the CD11b+ monocyte number. By having tested six increasing CRX601 doses, we are showing that the amount of 1 µg of CRX 601 is enough to trigger an increase of the monocyte count within the peripheral blood. A constant increase is noted until maximum response at 10 µg dose and the effect is down modulated at 20ug dose.

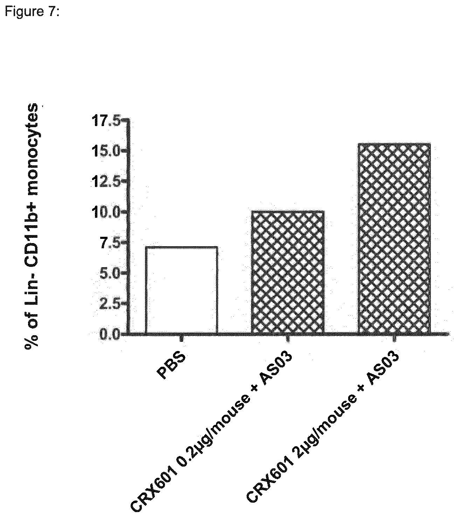

In Figure 7 we perform a dilution analysis of the CRX601 combined with a constant dose of AS03. Peripheral blood monocyte numbers were calculated following a single intramuscular injection of different doses of CRX601 (0.2µg to 20 µg).

### Methods:

Flow cytometry analysis:
Peripheral blood was drawn from C57BL/6 mice via cardiac puncture with lithium-heparin as anticoagulant, 24-Hour after injection of the TLR adjuvants. Red blood cell lysis was performed twice on pooled blood with Ammonium Chloride-based Buffer (Sigma, Steinheim, Germany) and cells were counted with the EasyCount™ System (Immunicon). After one washing step, 500,000 cells were incubated with Rat anti- Mouse CD16/CD32 (BD Fc Block™ by BD Biosciences) for 10 min. on ice and cells were further incubated for 30 min. with a combination of the following directly conjugated antibodies at their pre-determined optimal concentration as described by Mildner et al., 2007): PerCP labeled-Streptavidin, PE- Hamster anti-Mouse CD3, Rat anti-Mouse CD45R/B220, Rat anti-Mouse Ly-6G, Mouse anti-Mouse NK1.1 APC-conjugated Rat anti-Mouse CD11b, PE-Cy7-conjugated Hamster anti-Mouse CD11c, FITC- Rat Anti-Mouse Ly-6C (all from BD Biosciences) and Pacific Blue™ Rat anti-Mouse CD62L (BioLegend, San Diego, CA). Cells were finally washed three times and fixed for 15 min. with a 2% paraformaldehyde solution in PBS. Fluorescence minus one (FMO) controls were always included in the assays for fluorescent compensation setting.

Samples were acquired on a flow cytometer (BD FACSCanto II) and data analyzed with the FACSDiva software (BD Biosciences).

Monocytes were identified by their Side/Forward scatter properties and gated as CD3-/CD45R/B220-/Ly-6G-/NK1.1-(Lineage-)/CD11b+ cells. CD11b+ monocytes frequency was reported as a percentage of the total cells excluding debris.

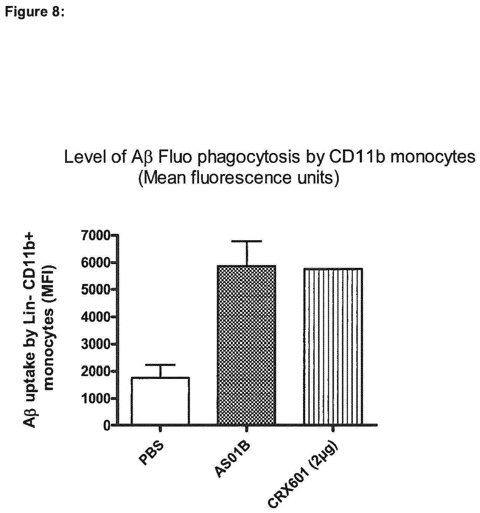

### Example 4, Figure 8

To begin to examine the function of the increase of monocytes in the peripheral blood, we examined the capacity of those monocytes to uptake Aβ42 in a test tube. In order to measure that phagocytic activity, we have used fluorescent HiLyteFluo Aβ42 (Anaspec Inc). Flow cytometry analysis demonstrated that the intramuscular injection of AS01B (mouse full dose) or CRX601 (2µg dose) trigger the monocytes to be able to uptake an higher amount of Aβ42 compared to a non adjuvanted mouse monocytes (PBS group)

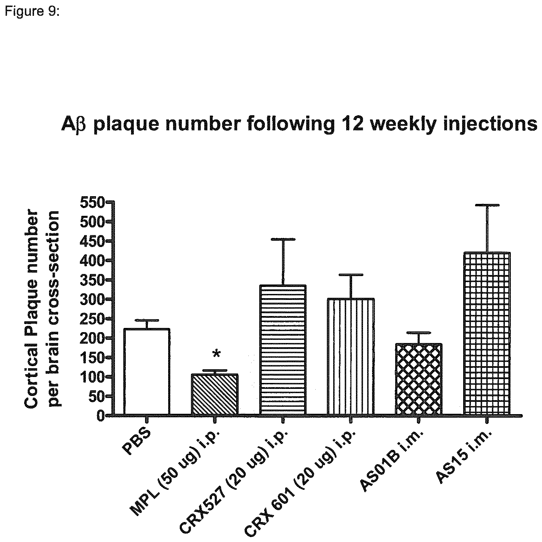

### Example 5, Figures 9 - 12

To determine whether TLR4 agonist free of endotoxins will improve the cognitive impairment and clearance of Aβ in APP/PS1 mice.

### Injections

Five groups of APP_{Swe}/PS1 mice have received one a week for a period of 12 weeks the following treatment:
- • Gr1:: APP_{Swe}/PS1 + NaCl 0.9 % n(=20), i.p. injections
- • Gr4:: APP_{Swe}/PS1 + CRX527 (20 µg/mouse, 130 µl, n=10), i.p. injections
- • Gr5:: APP_{Swe}/PS1 + CRX601 (20 µg/mouse, 130 µl, n=10), i.p. injections
- • Gr6:: APP_{Swe}/PS1 + 3D MPL (50 µg/mouse, 130 µl, n=10), i.p. injections
- • Gr8: APP_{Swe}/PS1 + AS15
- • Gr9:: APP_{Swe}/PS1 + AS01B (1/10th of human dose µg/kg, 50 µl, n=10), i.m. injections

| Group | Agonist | n = t₀ | n = t₁₂ |
|---|---|---|---|
| 1 | Saline | 20 | 19 |
| 4 | CRX527 | 10 | 9 |
| 5 | CRX601 | 10 | 10 |
| 6 | 3D-MPL | 10 | 9 |
| 8 | AS15 | 10 | 10 |
| 9 | AS01b | 10 | 10 |

### 1.2 BEHAVIORAL ANALYSES

### T-water maze

Mice were tested during the "light on" phase of the day. Behavioral experimenter was blinded to the genetic and treatment status of animals. To assess hippocampal-dependent spatial learning and memory, mice were trained in the T-water maze task. In this paradigm, we evaluate the mouse's ability to remember the spatial location of submerged platform. The T-maze apparatus (length of stem, 64 cm; length of arms, 30 cm; width, 12 cm; height of walls, 16 cm) was made of clear fiberglass and filled with water (23±1°C) at a height of 12 cm. A platform (11x11 cm) was placed at the end of the target arm and was submerged 1 cm below the surface. The acquisition phase allows to evaluate animals for left-right spatial learning. During the first two trials, platforms were placed on each arms of the maze to test the spontaneous turning preference of the mouse. After these two trials, the least chosen arm was reinforced by the escape platform. The mice were placed in the stem of the T-maze and choose to swim either left or right until they found the submerged platform and escape to it, to a maximum of 60 s. After reaching the platform, the mice remained on it for 20 s and then were immediately placed back in the maze. If the animals did not find the platform within this limit, they were gently guided onto it. Repeated trials were presented on the same day up to a maximum of 48 trials. A rest period of at least 10-15 min intervened between each block of 10 trials. A mouse was considered to have learned the task when it made no errors in a block of five consecutive trials. The reversal learning phase was then conducted 48 h later. During this phase, the same protocol was repeated, except that the mice were trained to find the escape platform on the opposite side to that on which they had learned on acquisition phase. The number of trials to reach the criterion (five of five correct choices made on consecutive trials) was measured as well as the latency to find the escape platform.

### Passive avoidance test

Based on the animal's natural tendency to prefer the dark environment, the animals were also evaluated in retention of non-spatial memory for one-trial passive avoidance task. The passive avoidance apparatus (Ugo Basile) was divided into two sections, one illuminated (the start compartment) and one dark (escape compartment). The floor of each compartment contained a grid, with only the dark compartment being electrified by a generator. On the training day, mice were placed into the lighted compartment for 60 s acclimation period. The guillotine door was then opened, and the latency to enter the dark side was recorded. Immediately after entering the dark compartment, the door was closed and an electric shock (0.5 mA for 2 s) was delivered. The mouse was kept in the dark compartment for 10 s before being returned to its home cage. On the next day, the mice were again placed in the light compartment, and the time, step through latency to enter the dark side, was measured for up to 300 s.

### Nesting behaviour

Thereafter, the nesting behaviour was used to test for changes in emotional status (e.g. apathy). Reduced nesting has been observed in hippocampal lesioned mice and mouse models of Alzheimer's disease (Deacon, 2006). Animals were individually housed in a cage containing sawdust and in which a 5 x 5 cm piece of cotton was introduced to allow nesting behaviour. One day later, the quality of the nest was determined according to a five-point scale as described by Deacon (2006): 1-Nestlet apparently untouched, 2-Nestlet partially torn up, 3-Nestlet mainly shredded but no apparent presence of nesting site, 4-Observable flat nest, 5-Observable (near) perfect nest.

### TISSUE ANALYSES

Mice were anesthetized under isofluorane and blood was drawn *via* cardiac puncture before head decapitation. Brains were rapidly removed from the skulls and placed in cold phosphate buffered saline (PBS) solution. Then hemibrains were separated and olfactory bulbs and cerebellum were removed. One hemibrain was rapidly frozen in liquid nitrogen and stored at -80°C for protein analysis. The other one was postfixed for 2-4 days in 4% paraformaldehyde (PFA), pH 9.5 at 4°C., and then placed in a PFA solution containing 10% sucrose overnight at 4°C. The frozen brains were mounted on a microtome (Reichert-Jung) and cut into 25-µm coronal sections. The slices were collected in cold cryoprotectant solution (0.05 M sodium phosphate buffer, pH 7.3, 30% ethylene glycol, and 20% glycerol) and stored at -20°C until immunocytochemistry or *in situ* hybridization histochemistry.

### Stereological analysis.

An observer who was blind to the treatment status of the material did all quantitative histological analyses. To count Aβplaques, sections of APP_{Swe}/PS1 mice were immunostained for Aβ (polyclonal mouse anti-Aβ 6E10, 1:3000; Covariance) as previously reported (Richard et al., 2008; Simard et al., 2006). Two sections were chosen for prefrontal cortex at +2.34 and +2.10 mm from the bregma according to a stereotaxic atlas (Paxinos and Franklin, second edition) and four sections for hippocampus/cerebral cortex at -1.70, -1.94, -2.46 and -2.92 mm. Unbiased stereological analysis was performed as described previously (Boissonneault et al., 2009; Richard et al., 2008; Simard et al., 2006). Briefly, the contours of the prefrontal cortex, the hippocampus and the cortex areas were traced as virtual overlay on the steamed images and areas were calculated. The area occupied by all Aβ-labeled plaques was determined. Real-time images (1600_1200 pixels) were obtained using a Nikon C80i microscope equipped with both a motorized stage (Ludl) and a MicrofireCCD color camera (Optronics). Such an apparatus was operated using the Stereo Investigator software designed by Microbrightfield. Both cortex and hippocampus areas were traced using a Cintiq 18S interactive pen display (Wacom).

*Protein extraction and detection of total A*β *levels by Western blot.*

Proteins from hemi-forebrains were extracted using a modified method of the procedure published by Lesné et al (Lesne et al., 2006). All manipulations were done on ice to minimize protein degradation. One hemi-forebrain was placed in a 1 ml syringe with a 20 G needle. 500 µl of buffer A (50 mM Tris-HCl pH 7.6, 0.01% NP-40, 150 mM NaCl, 2 mM EDTA, 0.1% SDS, 1 mM phenylmethylsulfonyl fluoride (PMSF), protease inhibitor cocktail) were added and 10 up and down were made to homogenize the tissue, followed by a 5 minutes centrifugation at 3 000 RPM at 4°C. The supernatant (extracellular proteins enriched fraction) was then collected and frozen at -80°C. The insoluble pellet was suspended in 500 µl TNT-buffer (Buffer B; 50 mM Tris-HCl pH 7.6,150 mM NaCl, 0.1 % Triton X-100, 1 mM PMSF, protease inhibitor cocktail), followed by a 90 minutes centrifugation at 13 000 RPM at 4°C. The supernatant (cytoplasmic proteins enriched fraction) was then collected and frozen at -80°C. The pellet was suspended in 500 µl buffer C (50 mM Tris-HCl pH 7.4, 150 mM NaCl, 0.5 % Triton X-100, 1 mM EGTA, 3 % SDS, 1 % deoxycholate, 1 mM PMSF, protease inhibitor cocktail) and incubated at 4°C, 50 RPM, for 1 hour. The samples were centrifuged for 90 minutes at 13 000 RPM and 4°C and the supernatant (membrane proteins enriched fraction) was collected and frozen at -80°C. Protein concentration of each fraction was determined using the Quantipro BCA assay kit (Sigma) according to the manufacturer protocol.

For total Aβ detection, 10-30 µg of extracellular, cytoplasmic and membrane protein fractions were separated on a precast 10-20% SDS polyacrylamide Tris-Tricine gel (Bio-Rad). Separated proteins were then transferred onto polyvinylidene fluoride (PVDF) membranes (PerkinElmer Life and Analytical Sciences) and detected by Western blotting. Blots were probed with a mouse anti-amyloid beta protein monoclonal antibody clone 6E10 (1:1000, Covariance) in 1 M Tris-HCl, pH 8.0, 5 M NaCl, 5% skim milk, and 0.05% Tween 20. Blots were visualized using anti-mouse secondary antibody tagged with horseradish peroxidase (1:1000; Jackson Immuno-Research) and enhanced chemiluminescence (PerkinElmer Life and Analytical Sciences). Membranes were stripped in 25 mM glycine-HCl, pH 2.0, containing 1% SDS to allow actin revelation using first a mouse actin antibody (MAB1501, 1:5000; Millipore Bioscience Research Reagents) and then a goat anti-mouse peroxidase conjugated secondary antibody (1:1000; Jackson ImmunoResearch).

Quantification was done by determining integrative density of the bands using a gel imaging system (scanner Agfa Arcus II;NIHlmage J software version 1.32j) and background values were removed. Optical values were normalized according to the actin loading control. Results are expressed as mean ± SEM.

### Results

Results for Aβ total plaque loading are shown in Figure 9. Aβ total plaque loading analyses reveal a significant difference between the PBS control group versus the 3D MPL group in term of Abeta plaque loading measurement by immunofluorescence. * indicates ANOVA, P=0.05 vs PBS group.

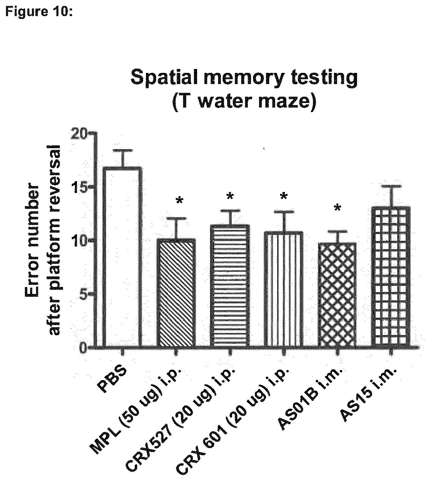

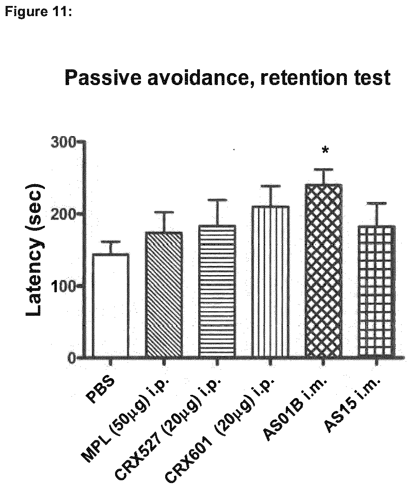

Results for behavioural analysis are provided in Figures 10 and 11.

In Fig 10 Twelve weekly injections of 3D MPL or CRX527 or CRX601 or AS01 B in APP/PS1 mouse model shows a spatial memory improvement compared to non-treated mice

In Fig 11 AS01 B treated animals exhibit a significant retention score compared to non-treated animals. Step through latencies were measured during the passive avoidance after the 12^{th} weekly injection. Data are expressed as mean (+/- SEM (One-way ANOVA)).

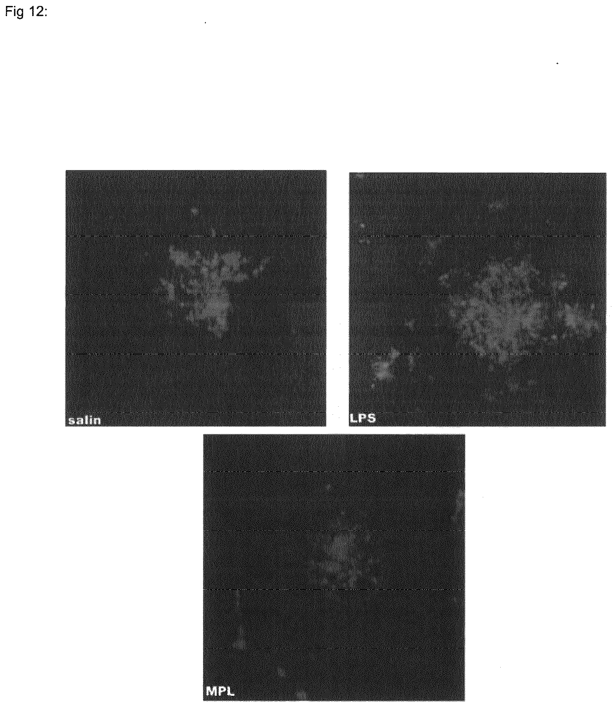

Results for brain histology are provided in Figure 12.

3D-MPL provides a statistically significant reduction in Amyloid beta plaque number.

3D-MPL, CRX 527, CRX 601 and AS01 B all provide significant improvements in behaviour as assessed by T water maze testing.

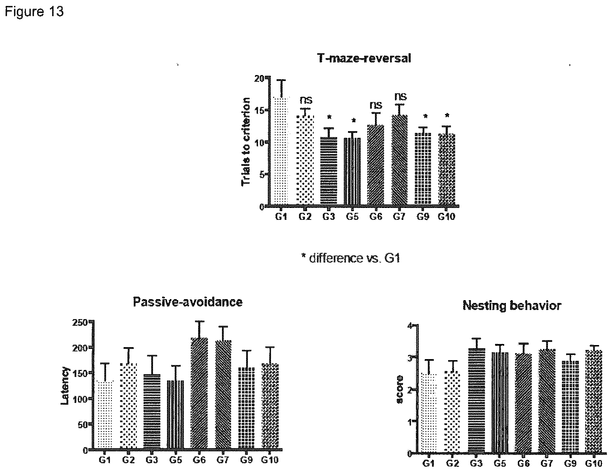

### Example 6, Figure 13

Further experiments were carried out using the following groups.
Gr 1: PBS i.m. - 12 x weekly, Negative control [n=10 (2 females]
Gr 2: CRX-601 i.m. (0.2 ug per mouse) [n=10 (2 females)]
Gr 3: CRX-601 i.m. (2 ug per mouse), 12 x weekly [n=10 (2 females)]
Gr 5: AS03-CRX601 (2ug dose for CRX601, 1/10 human dose for AS03, i.m. 12 x weekly [n=10 (2 females)]
Gr 6: AS01 B i.m (1/10 human dose), 12 x weekly [n=10 (2 females)]
Gr 7: AS01 B i.m (1/50 human dose), 12 x weekly [n=10 (2 females)]
Gr 9: 3D 3D MPL intra peritoneal (as it in Aim 1), 50ug per mouse, 12 x weekly [n=10 (2 females)]
Gr 10: 3D MPL i.m. (5 ug per mouse), 12 x weekly [n=10 (2 females)]

Results indicate that groups 3, 5, 9 and 10 were significantly improved in the T maze reversal test compared with group 1.

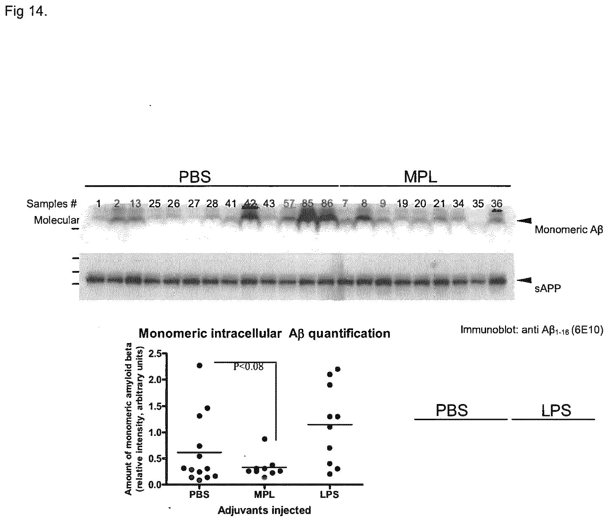

### Example 7 Fig 14

3D MPL and PBS were injected into mice, and the monomerisation status of intracellular amyloid beta was carried out by an immunoblot using Immunoblot: anti Aβ1-16 antibody. There was a reduction of monomeric Aβ in extra-cellular enriched fractions from brains of 3D MPL-injected mice.

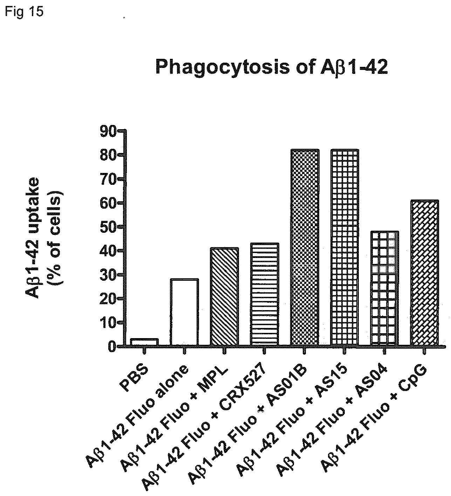

### Example 8 Figure 15

Phagocytosis of beta-amyloid 1-42 peptide by human microglial cells was observed after addition of a range of compounds of the invention. Most of the compounds used increase the percentage of phagocytosis of beta-amyloid 1-42 peptide by the human microglial cells. In this experiment, the highest increase of phagocytosis is observed with AS01B and AS15.

In figure 15, Human microglia cell line (CHME) is showing that their Aβ42 phagocytic activity is increased by the pre-incubation during 18 hrs of the cells with the adjuvants. Adjuvant dose for that in vitro phagocytosis assay was based on the MPL content (i.e. 2 ug per ml). Cpg 7909 adjuvant was purchased from Invivogen and used at 79 ug per ml. Aβ42 was used at 1ug per ml in DMEM complete media (Invitrogen).

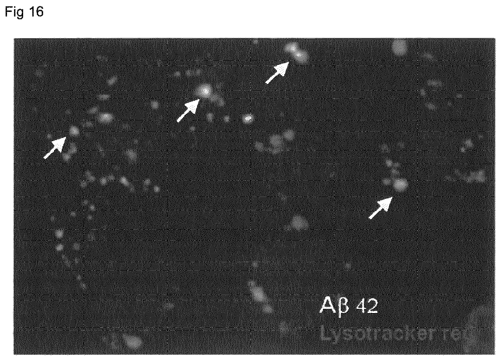

### Example 9 Figure 16

Human microglia cell were treated for 18 hrs with AS01 B having 1µg/ml of 3D MPL in the presence of 1µg/ml of HiLyteFluo Aβ1-42. Lysotracker red staining was performed and slides were mounted and co-stained with DAPI to show the nucleus (blue).

The representative picture of fluorescence microscopy of human microglia cell line shows the localization of Aβ1-42 within the lysosome after AS01 B treatment.

Human microglia cell line have more amyloid phagocytic activity following stimulation with lipid A containing adjuvants. The amyloid is targeted to the lysosome. Representative picture of previous experiment as described in Fig. 15. We have performed double immunofluorescence to detect Abeta within the lysosome compartment within the CHME human microglia cell line. CHMEcells were co-incubated with Abeta 42 fluorescent (HiLyte Fluo 488, Anaspec) at 1 ug per ml and MPL at 1 ug per ml. Lysotracker red reagent was purchased from Invitrogen and used as manufacturer recommendations.

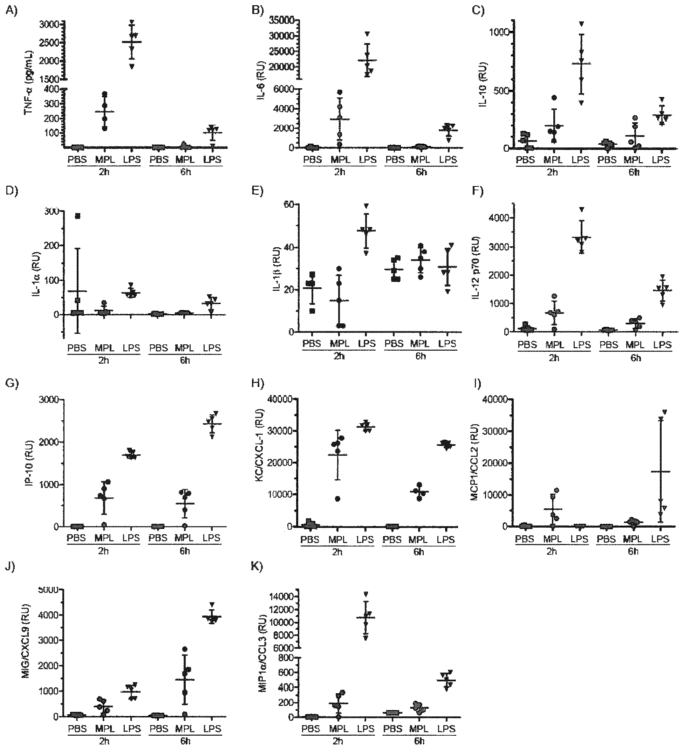

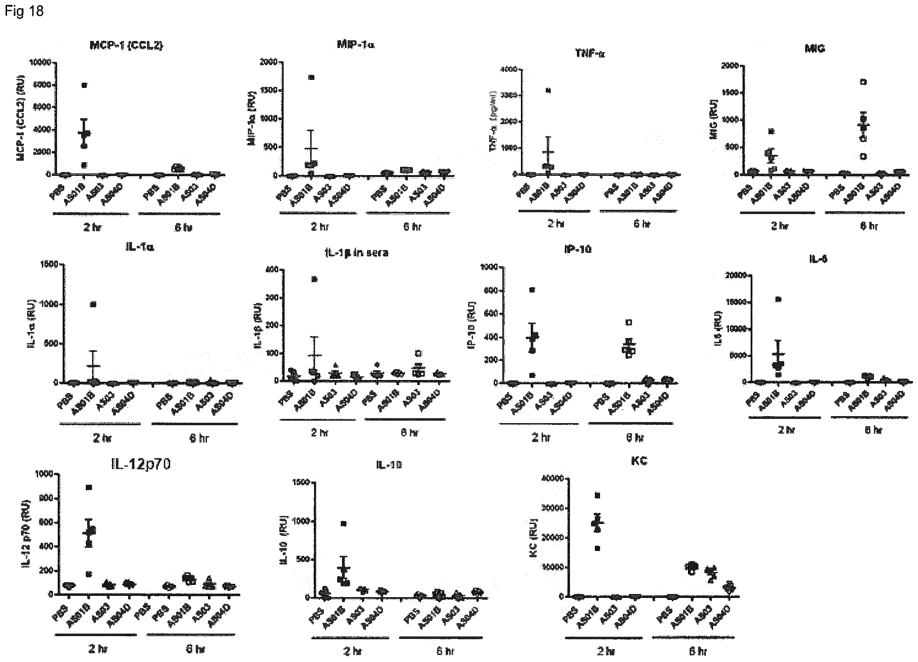

### Example 10: Figures 17 and 18

Microglia are often seen surrounding and at lower extend inside the Amyloid β plaques in Alzheimer's brains and trying to clear those plaques. This phenomenon of microglia activation might need to be regulated in a control manner to avoid the detrimental effect of activating too much the CNS immune cells. Strongest activators of microglia are the Toll-like receptor 4 agonists such as lipopolysaccharides (LPS). LPS provokes a rapid and strong innate activation of brain cells such as microglia and their precursors in the peripheral blood such as monocytes that come from bone marrow myeloid cells. However, LPS from E. coli, Salmonella and few other gram negative bacteria are strong endotoxins, are toxic and has been shown to exacerbate pre-existing neuropathology in mice when injected at the peripheral blood. LPS could not be used at clinical level because of high toxicity. Therefore, to avoid those detrimental effects, we evaluated herein a detoxified derivative molecule of lipopolysaccharide called 3D MPL (3-O-desacyl-4'-monophosphoryl lipid A) isolated from the Gram negative bacterium Salmonella minnesota R595 strain. We show that 3DMPL delivered by the intraperitoneal route promotes an attenuated cytokine profile compared to LPS, while it can activate the increase in phatocytic cells and phagocytosis. This suggests that 3DMPL is better suited to induce the activation of phagocytic cells, e.g. microglia cells, without provoking a burst of pro-inflammatory cytokine with the risk to exacerbate neurodegenerative lesions..

Similarly, we show that AS01B injected by the intramuscular route, a 3D MPL-containing liposomal formulation shows even more attenuated to similar biological activity on the cytokine production as 3DMPL, which suggest that it may used instead of MPL to achieve a comparable systemic innate activation.

### Results:

### Figure 17 - 3DMPL induces a low inflammatory response in mice

We measured several cytokines and chemokines in the sera of C57BL/6 mice 2 and 6 h following a single intraperitoneal injection of either MPL or LPS. We found that most of the cytokine and chemokine levels were increased in 3DMPL-injected mice, but these levels were substantially lower than those of LPS-treated animals (Fig. 17a to k). The levels of TNF-α and IL-6 were very high 2 h post LPS injection while a modest increase was observed in 3DMPL-injected mice but it was essentially abolished after 6 h. Interestingly, 2 h after the injection, the chemokines which are more related to monocytes and microglia activation such as CXCL-1 and CCL2 were modulated respectively to similar or higher levels in MPL-treated mice compared to the LPS group.

### Figure 18 - 3DMPL induces a low inflammatory response in mice

Innate cytokines profile from sera after either AS01B, AS03 or AS04D was injected into mice using the intramuscular route, samples taken at 2hr or 6hr post injection time point. Results are shown in relative units (RU or pg/ml) of various cytokines/chemokines in sera for PBS and LPS or MPL injected mice after 2 hours or 6 hours. N = 5 mice per group. The bars represent mean ± SEM.

### Example 11 - Monocyte analysis and counting after adjuvant injection in mice

TLR adjuvants were tested for their ability to stimulate peripheral monocytes

24-Hour after injection of the TLR adjuvants, peripheral blood was drawn from C57BL/6 mice via cardiac puncture with lithium-heparin as anticoagulant. Red blood cell lysis was performed twice on pooled blood with Ammonium Chloride-based Buffer (Sigma, Steinheim, Germany) and cells were counted with the EasyCount™ System (Immunicon). After one washing step, 500,000 cells were incubated with Rat anti- Mouse CD16/CD32 (BD Fc Block™ by BD Biosciences) for 10 min. on ice and cells were further incubated for 30 min. with a combination of the following directly conjugated antibodies at their pre-determined optimal concentration as described by Mildner et al., 2007): PerCP labeled-Streptavidin, PE-Hamster anti-Mouse CD3, Rat anti-Mouse CD45R/B220, Rat anti-Mouse Ly-6G, Mouse anti-Mouse NK1.1 APC-conjugated Rat anti-Mouse CD11b, PE-Cy7-conjugated Hamster anti-Mouse CD11c, FITC-Rat Anti-Mouse Ly-6C (all from BD Biosciences) and Pacific Blue™ Rat anti-Mouse CD62L (BioLegend, San Diego, CA). Cells were finally washed three times and fixed for 15 min. with a 2% paraformaldehyde solution in PBS. Fluorescence minus one (FMO) controls were always included in the assays for fluorescent compensation setting. Samples were acquired on a flow cytometer (BD FACSCanto II) and data analyzed with the FACSDiva software (BD Biosciences). Monocytes were identified by their Side/Forward scatter properties and gated as CD3-/CD45R/B220-/Ly-6G-/NK1.1-(Lineage-)/CD11b+ cells. CD11 b+ monocytes frequency was reported as a percentage of the total cells excluding debris.

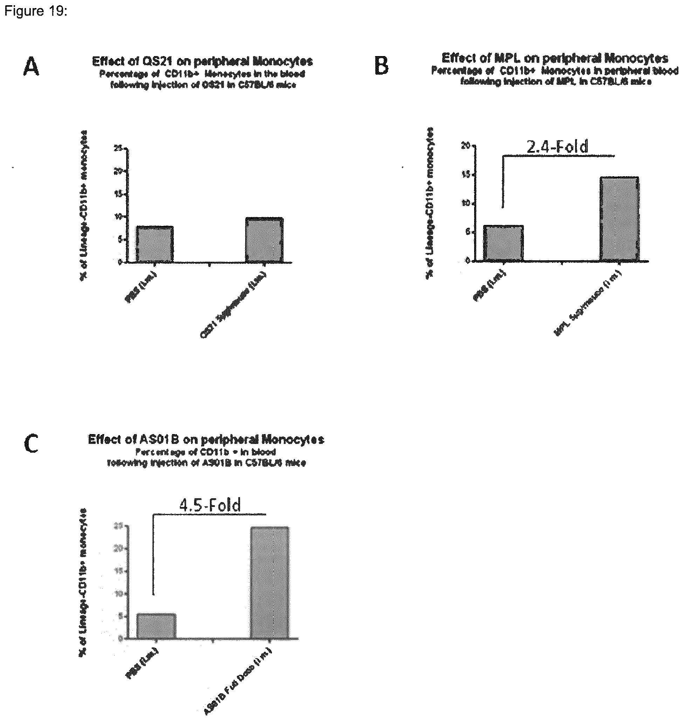

### Example 11 (Figure 19)

Different compositions were used to test for stimulation of peripheral monocytes.

AS01 B is better than 3D MPL alone in upregulating monocytes.

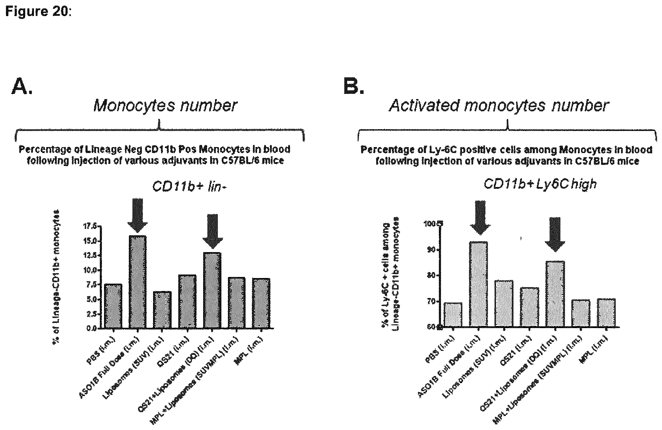

### Example 12 Figure 20

Samples were taken after 24 hrs of the intra muscular injection of each single component as shown in the Figure 20.

24-Hour after injection of each immunomodulator or adjuvants. Peripheral blood was drawn from C57BL/6 mice via cardiac puncture with lithium-heparin as anticoagulant, Red blood cell lysis was performed twice on pooled blood with Ammonium Chloride-based Buffer (Sigma, Steinheim, Germany) and cells were counted with the EasyCount™ System (Immunicon). After one washing step, 500,000 cells were incubated with Rat anti- Mouse CD16/CD32 (BD Fc Block™ by BD Biosciences) for 10 min. on ice and cells were further incubated for 30 min. with a combination of the following directly conjugated antibodies at their pre-determined optimal concentration as described by Mildner et al., 2007): PerCP labeled-Streptavidin, PE- Hamster anti-Mouse CD3, Rat anti-Mouse CD45R/B220, Rat anti-Mouse Ly-6G, Mouse anti-Mouse NK1.1 APC-conjugated Rat anti-Mouse CD11b, PE-Cy7-conjugated Hamster anti-Mouse CD11c, FITC- Rat Anti-Mouse Ly-6C (all from BD Biosciences) and Pacific Blue™ Rat anti-Mouse CD62L (BioLegend, San Diego, CA). Cells were finally washed three times and fixed for 15 min. with a 2% paraformaldehyde solution in PBS. Fluorescence minus one (FMO) controls were always included in the assays for fluorescent compensation setting. Samples were acquired on a flow cytometer (BD FACSCanto II) and data analyzed with the FACSDiva software (BD Biosciences). Monocytes were identified by their Side/Forward scatter properties and gated as CD3-/CD45R/B220-/Ly-6G-/NK1.1-(Lineage-)/CD11b+ cells. CD11 b+ monocytes frequency was reported as a percentage of the total cells excluding debris.

AS01B and QS21 + liposome show most significant monocyte number increase (panel A) and the monocyte activation state (Ly6C high) (panel B) after 24hrs in the C57BL/6 mouse peripheral blood following a single injection.

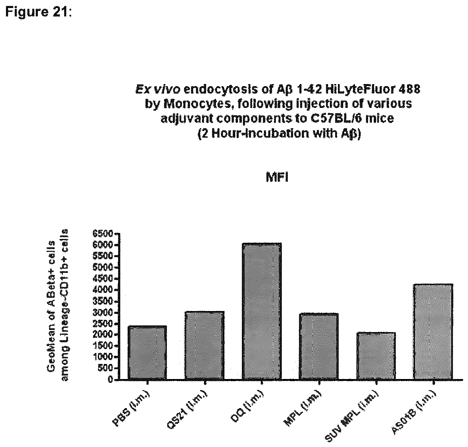

### Example 13 Figure 21

We examined the capacity of monocytes to uptake Aβ42 in a test tube. In order to measure that phagocytic activity we have used fluorescent HiLyteFluo Aβ42 (Anaspec Inc). Flow cytometry analysis demonstrated that the intramuscular injection of DQ (QS21 + liposome) triggers the monocytes to be able to uptake a higher amount of Aβ42 compared to PBS or QS21 (5 µg) injected mice or liposomal MPL (5 µg of SUV MPL) or MPL itself at the 5 µg dose used herein. This is suggests to us that QS21 does not promote the Aβ uptake and the liposomal + QS21 is necessary to promote that last described effect.

All experiments and assays were performed in accordance with the Canadian Council on Animal Care (CCAC) guidelines for animal experimentation. Eight weeks female C57BL/6 mice were obtained from Charles-Rivers laboratories (St-Constant, Quebec). The APP-PS1 mouse model was obtained from Jackson laboratories, stock 5866 (Savonenko et al., 2005). Intramuscular injections in mice were performed on either the gastrocnemius anterior in 50 or 25 µL depending on the experiments. Intravenous injections (100 µL) were performed in the tail vein. The adjuvant composition was as follow:
Adjuvant composition:
   For the AS01B, AS03 and AS15. The mouse dose is equal of the 1/10 human dose.

AS01 B is composed of liposomes containing 3D-MPL and QS21 The mouse dose of AS01 B contains 5 µg of MPL 3D co-formulated in neutral liposome, 5 µg of QS21. Those doses are per mouse and were injected using the intramuscular route (i.m.) 25 µl per mouse of AS01B + 25 ul of PBS (phosphate buffer saline) or 25 µl of the appropriate peptide.

Ex vivo uptake assay of Ab42

Preparation of cells: Peripheral blood was drawn from C57BL/6 mice via cardiac puncture with lithium-heparin as anticoagulant, 24-Hour after injection of the adjuvants used herein. Red blood cell lysis was performed twice on pooled blood with Ammonium Chloride-based Buffer (Sigma, Steinheim, Germany) and cells were counted with the EasyCount™ System (Immunicon).

Cell stimulation/Aβ phagocytosis: cells were seeded at 106 cells/mL onto a 24-well tissue culture plate and stimulated for 2 or 24 h in the presence or absence of 1 µg/ml of Aβ1-42 HiLyte Fluor™488 (Anaspec, Fremont, CA), which was pre-incubated or not for 1h with 1 µg/ml of anti-amyloid β antibodies (e.g. anti-Aβ1-17 IgG1, clone 6E10, Signet Laboratories, Dedham, MA) or with purified IgG from mouse serum (Sigma), as control.

FACS analysis: cells were harvested after incubation with fluorescent Aβ peptide with Trypsin/EDTA and cold PBS and washed three times. 500,000 cells were incubated in 96-well plate for 10 min. on ice in the presence of Rat anti- Mouse CD16/CD32 (clone 2.4G2 - BD Fc Block™) and further stained for 30 min. with a combination of the following directly conjugated antibodies at their pre-determined optimal concentration: PE- Hamster anti-Mouse CD3 (clone 145-2C11), Rat anti-Mouse CD45R/B220 (clone RA3-6B2), Rat anti-Mouse Ly-6G (clone 1A8), Mouse anti-Mouse NK1.1 (clone PK136), APC-conjugated Rat anti-Mouse CD11b (clone M1/70), PE-Cy7-conjugated Hamster anti-Mouse CD11c (clone HL3), (all from BD PharMingen). Cells were finally washed twice and fixed for 15 min. with a 2% paraformaldehyde solution in PBS. FMOs controls were always included in the assays.

Samples were acquired on a flow cytometer (BD FACSCanto II) and data analyzed with the FACSDiva software (BD Biosciences).

Monocytes were identified by their Side/Forward scatter properties, excluding debris and gated as CD3-/CD45R/B220-/Ly-6G-/NK1.1-(Lineage-)/CD11b+ cells. Aβ uptake was assessed by reporting the percentage and Mean Fluorescence Intensity (GeoMean) of positive HiLyte fluor488 Aβ1-42 cells among gated monocytes.

## Claims

1. A method of preventing and/or reducing amyloid deposition in a subject comprising treatment of a subject with a composition comprising a TLR4 agonist free of endotoxin.

2. A composition comprising a TLR4 agonist free of endotoxin for preventing and/or reducing amyloid deposition in a subject.

3. Use of a composition comprising a TLR4 agonist free of endotoxin in the manufacture of a medicament for preventing and/or reducing amyloid deposition in a subject.

4. A method of preventing and/or reducing Alzheimer's disease in a subject comprising treatment of a subject with a composition comprising a TLR4 agonist free of endotoxin .

5. A TLR4 agonist free of endotoxin for preventing and/or reducing Alzheimer's disease.

6. Use of a TLR4 agonist free of endotoxin in the manufacture of a medicament for preventing and/or reducing Alzheimer's disease.

7. Use or method according to any claims 1-6 as assessed by improvement of spatial memory in a treated subject.

8. Use or method according to any claims 1-6 as assessed by both amyloid beta plaque reduction and a behavioural test.

9. Use or method according to claim 8 wherein the treatment is with 3D-MPL or MPL.

10. A method or use according to claim 9 wherein the MPL or 3D-MPL is combined with an oil in water emulsion.

11. A method or use according to any preceding claim wherein the composition comprises 3D MPL, QS21 and a liposome, such as AS01 B.

12. A method or use according to any preceding claim wherein the composition does not contain beta amyloid or a fragment or mimotope thereof.

13. A method or use according to any preceding claim wherein the composition consists of, or consists essentially of an aminoalkyl glucosaminide phosphate ("AGP"), 3D-MPL, AS01 B or an AGP in combination with an oil in water emulsion.

14. A pharmaceutical composition consisting of, or consisting essentially of, an aminoalkyl glucosaminide phosphate ("AGP"), 3D-MPL, AS01B or an AGP in combination with an oil in water emulsion.

15. Use of TLR4 agonist free of endotoxin to increase the activation of microglia within the brain.

16. A method or use according to any preceding claim wherein preventing and/or reducing amyloid deposition or Alzheimer's disease is by increasing the phagocytosis of amyloid beta.
